# EUROPEAN PATENT APPLICATION

(11) **EP 4 745 162 A1**
(43) Date of publication of application: **20.05.2026**
(21) Application number: 24864718.2
(22) Date of filing: 12.09.2024
(51) Int. Cl.: C07K 19/00, C12N 5/10, C12N 15/62, C12N 15/86, A61K 39/00, A61K 39/395, A61K 38/20, A61P 35/00, A61P 35/02

(54) **IMMUNE CELL EXPRESSING FUSION PROTEIN, AND EXPRESSION VECTOR THEREOF, USE THEREOF AND PREPARATION METHOD THEREFOR**

(30) Priority: 13.09.2023 CN 202311180600
(71) Applicant: CD (SUZHOU) BIOPHARMA CO., LTD., Suzhou, Jiangsu 215000 (CN)
(72) Inventor: LI, Ying, Suzhou, Jiangsu 215000 (CN); WANG, Shengdian, Suzhou, Jiangsu 215000 (CN); ZHAO, Jitao, Suzhou, Jiangsu 215000 (CN); YANG, Naibo, Suzhou, Jiangsu 215000 (CN); CHENG, Xiangrui, Suzhou, Jiangsu 215000 (CN); HE, Qianqian, Suzhou, Jiangsu 215000 (CN)
(74) Representative: Santarelli
(86) International application number: PCT/CN2024/118641
(87) International publication number: WO 2025/056004

(57) **Abstract**

Disclosed are a fusion protein including an anti-programmed cell death protein 1 (PD-1) single-chain variable fragment (scFv) and interleukin-21 (IL-21), and an immune cell capable of expressing and secreting the fusion protein, as well as an expression vector, a use, and a preparation method thereof.

## Description

### CROSS-REFERENCE TO RELATED APPLICATIONS

The present application claims priority to Chinese Patent Application No. 202311180600.6, filed on September 13, 2023, titled "IMMUNE CELL EXPRESSING FUSION PROTEIN AND EXPRESSION VECTOR, USE AND PREPARATION METHOD THEREOF", and the entire contents of which are incorporated herein by reference.

### Technical Field

The present application relates to the technical field of immune cells, and in particular to an immune cell and an expression vector, a use and a preparation method thereof.

### Background of the Invention

Chimeric antigen receptor T (CAR-T) cell therapy, based on T cells genetically modified with a chimeric antigen receptor (CAR), involves introducing exogenous, artificially designed CAR genes capable of recognizing antigens on a tumor cell surface into the T cells for genetic modification, thereby generating CAR-expressing T cells. These cells are then expanded to a large scale in vitro and reinfused into patients for treatment. A CAR molecule primarily consists of three parts: an extracellular tumor antigen binding domain, a transmembrane domain, and an intracellular signal domain. The extracellular antigen binding domain is typically derived from a single chain variable fragment (scFv) against the antigens on the tumor cell surface; and the intracellular signal domain mainly includes intracellular signaling domains of a CD3ζ chain of a T-cell receptor (TCR) and a co-stimulatory molecule such as CD28 or 4-1BB. When the CAR on CAR-T cells specifically binds to the antigens on the tumor cell surface, the T cells are activated by the activation signals transduced via the intracellular domains, thereby exerting killing and anti-tumor effects of the T cells. Currently, in clinical treatments and the majority of clinical trials for hematologic malignancies, second-generation CAR-T cell therapy technology including the intracellular signaling domain of the co-stimulatory molecule such as CD28 or 4-1BB remains the mainstream approach.

To address the issues of limited overall efficacy and high relapse rate of the current CAR-T cell therapies, extensive research and development have been carried out on CAR-T cell technology. Like endogenous T cells, CAR-T cells also upregulate the expression of the immune checkpoint programmed cell death protein 1 (PD-1) upon antigenic stimulation. The binding of the PD-1 on the surface of the CAR-T cells to the programmed death factor ligand 1 (PD-L1) expressed by tumor cells or tissues will inhibit the anti-tumor function of the CAR-T cells. Thus, one research and development strategy to enhance the function of the CAR-T cells is to inhibit or eliminate the inherent PD-1 signals in the CAR-T cells. For example, PD-1 expression on CAR-T cells can be inhibited by expressing short hairpin ribonucleic acids (shRNAs) targeting PD-1. PD-1 deficient CD19 CAR-T cells generated via clustered regularly interspaced short palindromic repeats (CRISPR)/Cas9 technology have already advanced to clinical trials. Cherkassky et al. constructed a dominant negative receptor (DNR) consisting of an extracellular domain of PD-1 and a CD8 transmembrane domain, and CD19 CAR-T cells co-transducing the PD-1 DNR demonstrated enhanced anti-tumor effect both in vitro and in vivo. In another approach, a PD-1/CD28 chimeric switch receptor was constructed by fusing an extracellular domain of PD-1 with transmembrane and intracellular domains of CD28, which transfected CAR-T cells to enable the conversion of PD-1 inhibitory signals into CD28 activation signals. Additional strategies involved incorporating a gene sequence capable of secreting an anti-PD-L1 or anti-PD-1 scFv based on the structure of second-generation CAR-T cells, enabling the CAR-T cells to not only kill tumor cells during contact with the tumor cells, but also locally secrete the scFv to block the binding of PD-1 to PD-L1, thereby enhancing the killing activity of the CAR-T cells against tumor cells. However, the tumor microenvironment is a complex immunosuppressive network composed of diverse immunosuppressive signals and mechanisms, the above strategies merely block or eliminate the PD-1 inhibitory signals of the CAR-T cells, and therefore cannot substantially improve the effector function of the CAR-T cells.

Other strategies focus on enhancing the innate and adaptive immunity of a body to improve the anti-tumor efficacy of CAR-T cells. These include engineering CAR-T cells to express an immunostimulant RN7SL1, immune-stimulating molecules IL-12, IL-18, and CD40L, and a chemokine, etc. By delivering these immune-stimulating molecules to tumor tissues, the tumor microenvironment can be reshaped, thereby enhancing the efficacy of the CAR-T cells. Additionally, to improve the proliferation and persistence of the CAR-T cells in vivo, CAR-T cells that constitutively co-express cytokines such as IL-2, IL-7, IL-15, or IL-21 have been constructed. However, the immunostimulants, cytokines, or chemokines generated by these CAR-T cells can enter systemic circulation, leading to severe adverse effects. Consequently, these CAR-T cell strategies remain at the early research stage and have not progressed into clinical trials.

Immune cell therapy for treating hematological malignancies represented by CD19 CAR-T cells and B-cell maturation antigen (BCMA) CAR-T cells has seen six approved products in the United States and five in China, which are used for treating B-cell lymphoblastic leukemia, lymphoma, and multiple myeloma. However, current CAR-T cell therapy technology still faces numerous challenges, for example:
(1) The overall efficacy of the CAR-T cell therapy remains to be improved.

The complete remission rate of the approved CD19 CAR-T cell therapies for acute B-cell lymphoblastic leukemia exceeds 80%, whereas the complete remission rate for B-cell lymphoma is around 60%, and is even lower for large lymphoma (with a diameter greater than 7 cm), and the therapeutic effect on other CD19-positive B-cell tumors is still unsatisfactory. For BCMA CAR-T cell therapies, the complete remission rate for multiple myeloma ranges from 60% to 80%. Moreover, all CAR-T cell therapies exhibit poor efficacy against brain tumors, and therefore patients with tumors that have invaded the brain are often excluded from the CAR-T cell therapy.

CAR-T cell therapies for solid tumors remain at early research and development stages, with unsatisfactory efficacy. One major factor is the high heterogeneity of tumor cells, that is, the expression levels of the tumor target molecules vary across tumor cells, and some tumors even lack expression entirely. Furthermore, the CAR-T cell therapy will further induce downregulation or loss of the expression of target molecules on tumor cells, resulting in escape of tumor cells from recognition by CAR-T cells. Additionally, the immunosuppressive microenvironment of the solid tumors inhibits the function of CAR-T cells that infiltrate the tumors and impairs the effective expansion of CAR-T cells.

(2) High tumor relapse rates of the approved CAR-T cell therapies.

Although the approved CD19 and BCMA CAR-T cell therapies have achieved relatively high remission rates in acute B-cell lymphoblastic leukemia and multiple myeloma, nearly half of the patients who have achieved remission experience relapse within 1 to 2 years. Loss or downregulation of tumor target antigens, constantly insufficient expansion of the CAR-T cells, or inability to persist long-term in vivo are key contributors for relapse after the CAR-T cell therapy. Among the patients undergoing CD19 CAR-T cell therapy, approximately 50% of children and young adults with relapsed acute B-cell lymphoblastic leukemia (B-ALL) have CD19 loss; and approximately 30% of patients with relapsed lymphoma also have CD19 loss, and another 30% have decreased CD19 expression. Antigen-negative tumor relapse primarily results from antigen loss. Even if antigens are not completely lost, immunoregulation reducing antigen expression or density is sufficient to allow the tumor cells to escape. Antigen-positive tumor relapse is caused by the inability of CAR-T cells to persist long-term in vivo.

In summary, existing technologies lack mature CAR-T cell therapy, which results in poor tumor treatment efficacy and a high propensity for tumor relapse.

### Summary of the Invention

To address at least one of the aforementioned technical problems, an objective of the present application is to provide a fusion protein including an anti-PD-1 scFv and interleukin-21 (IL-21), and an immune cell capable of expressing and secreting the fusion protein, as well as an expression vector, a use and a preparation method thereof. The immune cell of the present application can express and secrete the fusion protein including the anti-PD-1 scFv and the IL-21, thereby significantly enhancing the anti-tumor efficacy of the immune cell and the inherent anti-tumor T cell responses in a body.

The present application is achieved as follows:
In a first aspect, the present application provides a fusion protein (PD-1Ab21) including an anti-PD-1 scFv and IL-21.

In a preferred embodiment of the present application, the anti-PD-1 scFv includes a heavy chain variable region and a light chain variable region, where the heavy chain variable region includes heavy chain complementarity-determining region 1 (HCDR1), HCDR2, and HCDR3, which include or consist of amino acid sequences of SEQ ID No.69, SEQ ID No.70, and SEQ ID No.71, respectively; the light chain variable region includes light chain complementarity-determining region 1 (LCDR1), LCDR2, and LCDR3, which include or consist of amino acid sequences of SEQ ID No.72, SEQ ID No.73, and SEQ ID No.74, respectively; and the IL-21 includes or consists of an amino acid sequence of SEQ ID No.16.

In the present application, the HCDRs of the heavy chain variable region and the LCDRs of the light chain variable region are determined according to Kabat.

In a preferred embodiment of the present application, the heavy chain variable region of the anti-PD-1 scFv can include or consist of an amino acid sequence of SEQ ID No. 8, and the light chain variable region can include or consist of an amino acid sequence of SEQ ID No.12.

In a preferred embodiment of the present application, the heavy chain variable region and the light chain variable region of the anti-PD-1 scFv can be linked together via linker1, which includes or consists of an amino acid sequence of SEQ ID No. 10.

In a preferred embodiment of the present application, the IL-21 can include or consist of an amino acid sequence of SEQ ID No. 16.

In a preferred embodiment of the present application, the anti-PD-1 scFv and the IL-21 are linked together via linker2 to form the fusion protein. Exemplarily, the linker2 can include or consist of an amino acid sequence of SEQ ID No.14.

In an alternative embodiment, a C-terminus of the fusion protein further includes a His tag. In a specific embodiment, the His tag in the fusion protein is linked to the C-terminus via linker 3. Exemplarily, the linker3 can include or consist of an amino acid sequence of SEQ ID. No.18.

In a preferred embodiment of the present application, the fusion protein includes or consists of an amino acid sequence of SEQ ID. No. 2 or SEQ ID. No.4. Exemplarily, a coding nucleotide sequence of SEQ ID No. 2 is shown in SEQ ID No. 1; and a coding nucleotide sequence of SEQ ID No.4 is shown in SEQ ID No. 3.

In a second aspect, the present application provides an immune cell, where the immune cell is capable of expressing and secreting the fusion protein as described in the first aspect.

In a preferred embodiment of the present application, the immune cell specifically recognizes at least one of a hematologic malignancy, a lung cancer, a breast cancer, a liver cancer, a pancreatic cancer, a renal cancer, a prostate cancer, an ovarian cancer, a gastrointestinal tumor, a brain tumor, a neuroendocrine tumor, a bone tumor, and a soft tissue tumor.

In a preferred embodiment of the present application, the immune cell is selected from at least one of CAR-T cells, TCR-T cells, chimeric antigen receptor natural killer (CAR-NK) cells, and tumor-infiltrating lymphocytes (TILs).

In a preferred embodiment of the present application, the immune cell not only exerts its own anti-tumor efficacy, but also enhances inherent anti-tumor immune responses in a body.

In a third aspect, the present application provides a viral vector, where the viral vector is capable of expressing the fusion protein as described in the first aspect.

In a preferred embodiment of the present application, after the viral expression vector infects a cell, it causes the cell to secrete the fusion protein as described in the first aspect.

In a fourth aspect, the present application provides an immune cell expression vector, where the immune cell expression vector includes an expression plasmid targeting a specific antigen, a porcine teschovirus-1 2A peptide (P2A) sequence fragment, and a coding sequence fragment of the fusion protein as described in the first aspect; and
optionally, the immune cell expression vector further includes a His tag; where
the expression plasmid targeting a specific antigen includes an extracellular domain, a transmembrane domain, and an intracellular domain;
the extracellular domain includes a CD8α signal peptide fragment, a specific antibody fragment, and a CD8α hinge region fragment;
the transmembrane domain includes a CD8α transmembrane region fragment; and
the intracellular domain includes a 4-1BB signaling domain fragment and a CD3ζ signaling domain fragment.

In a preferred embodiment of the present application, an amino acid sequence of the fusion protein as described in the first aspect is shown in SEQ ID No.2 or SEQ ID No.4, and a complementary deoxyribonucleic acid (cDNA) sequences of its coding sequence fragment is shown in SEQ ID No.1 or SEQ ID No.3, respectively.

In a preferred embodiment of the present application, the specific antibody fragment in the aforementioned immune cell expression vector includes a specific scFv targeting CD19, and/or a specific scFv targeting BCMA, and/or a specific antibody fragment targeting a Her2/neu tumor antigen, and/or a specific antibody fragment targeting other tumor antigens.

In a preferred embodiment of the present application, a nucleotide sequence and an amino acid sequence of a light chain variable region of the specific scFv targeting CD19 are shown in SEQ ID No.25 and SEQ ID No.26, respectively, and a nucleotide sequence and an amino acid sequence of a heavy chain variable region are shown in SEQ ID No.29 and SEQ ID No.30, respectively.

In a specific embodiment of the present application, the light chain variable region and the heavy chain variable region of the specific scFv targeting CD19 are linked together via linker4. Exemplarily, a nucleotide sequence and an amino acid sequence of the linker4 can be shown in SEQ ID No.27 and SEQ ID No.28, respectively.

In an exemplary embodiment of the present application, a nucleotide sequence and an amino acid sequence of the specific scFv targeting CD19 are shown in SEQ ID No.31 and SEQ ID No.32, respectively.

In a preferred embodiment of the present application, a nucleotide sequence and an amino acid sequence of a light chain variable region of the specific scFv targeting BCMA are shown in SEQ ID No.47 and SEQ ID No.48, respectively, and a nucleotide sequence and an amino acid sequence of a heavy chain variable region are shown in SEQ ID No.51 and SEQ ID No.52, respectively.

In a specific embodiment of the present application, the light chain variable region and the heavy chain variable region of the specific scFv targeting BCMA are linked together via linker5. Exemplarily, a nucleotide sequence and an amino acid sequence of the linker5 can be shown in SEQ ID No.49 and SEQ ID No.50, respectively.

In an exemplary embodiment of the present application, a nucleotide sequence and an amino acid sequence of the specific scFv targeting BCMA are shown in SEQ ID. 45 and SEQ ID No.46, respectively.

In a preferred embodiment of the present application, a nucleotide sequence and an amino acid sequence of a light chain variable region of the specific scFv targeting Her2 are shown in SEQ ID No.59 and SEQ ID No.60, respectively, and a nucleotide sequence and an amino acid sequence of a heavy chain variable region are shown in SEQ ID No.63 and SEQ ID No.64, respectively.

In a specific embodiment of the present application, the light chain variable region and the heavy chain variable region of the specific scFv targeting Her2 are linked together via linker6. Exemplarily, a nucleotide sequence and an amino acid sequence of the linker6 can be shown in SEQ ID No.61 and SEQ ID No.62, respectively.

In an exemplary embodiment of the present application, a nucleotide sequence and an amino acid sequence of the specific scFv targeting Her2 are shown in SEQ ID No.57 and SEQ ID No.58, respectively.

In a fifth aspect, the present application provides a use of an immune cell in treating a B-cell leukemia, a B-cell lymphoma, a myeloma, a Her2/neu⁺ breast cancer, and an advanced B-cell malignancy, where the immune cell is the immune cell as described in the second aspect.

In a sixth aspect, the present application provides a treatment method of a B-cell leukemia, a B-cell lymphoma, a myeloma, a Her2/neu⁺ breast cancer, and an advanced B-cell malignancy, where a therapeutically effective amount of the immune cell as described in the first aspect is administered to a subject.

In a seventh aspect, the present application provides a preparation method of an immune cell, including steps of:
constructing a coding gene of the fusion protein as described in the first aspect;
constructing an immune cell expression vector expressing the fusion protein as described in the first aspect;
performing virus packaging on the immune cell expression vector to obtain viral particles; and
preparing the immune cell with the viral particles.

In a preferred embodiment of the present application, in the preparation method of the aforementioned immune cell, the step of constructing a coding gene of the fusion protein as described in the first aspect includes:
selecting a humanized PD-1 antibody and linking a heavy chain variable region with a light chain variable region together via Linker1 to obtain a anti-PD-1 scFv targeting specific cells; and
linking the anti-PD-1 scFv targeting specific cells with a cDNA sequence of IL-21 cytokine via Linker2 and performing DNA fragment synthesis to obtain the coding gene of the fusion protein as described in the first aspect.

In a preferred embodiment of the present application, in the preparation method of the aforementioned immune cell, the step of constructing an immune cell expression vector expressing the fusion protein as described in the first aspect includes:
constructing an expression plasmid targeting a specific antigen with a lentiviral vector pCDH, where the expression plasmid targeting a specific antigen includes an extracellular domain, a transmembrane domain, and an intracellular domain;
the extracellular domain includes a CD8α signal peptide fragment, a specific antibody fragment, and a CD8α hinge region fragment;
the specific antibody fragment includes a specific scFv targeting CD19, and/or a specific scFv targeting BCMA, and/or a specific antibody fragment targeting a Her2/neu tumor antigen, and/or a specific antibody fragment targeting other tumor antigens;
the transmembrane domain includes a CD8α transmembrane region fragment; and
the intracellular domain includes a 4-1BB signaling domain fragment and a CD3ζ signaling domain fragment;
linking a coding sequence fragment of the fusion protein as described in the first aspect downstream of a coding fragment of the expression plasmid targeting a specific antigen via a P2A sequence fragment; and
optionally, attaching a His tag to a C-terminus of an entire sequence to obtain the immune cell expression vector expressing the fusion protein as described in the first aspect.

Exemplarily, nucleotide sequences and amino acid sequences of the specific scFv targeting CD19, the specific scFv targeting BCMA, and the specific antibody fragment targeting a Her2/neu tumor antigen are as described above.

In a preferred embodiment of the present application, in the preparation method of the aforementioned immune cell, the step of performing virus packaging on the immune cell expression vector to obtain viral particles includes:
performing the virus packaging with a calcium phosphate transfection method;
16 to 20 hours before transfection, selecting 293T cells for subculture and inoculating 4×10⁶ 293T cells into each cell culture dish;
20 to 24 hours post the inoculation, observing the subculture of the 293T cells and performing the transfection when a coverage rate of the 293T cells reaches 50%; where
the transfection method includes: preparing a transfection reagent including solution A and solution B, where the solution A includes 35 µg of plasmids (15 µg of CAR expression vector plasmids, 7.5 µg of a helper plasmid Rev-response element (RRE), 6 µg of a regulator of expression of viral proteins (REV), and 6 µg of vesicular stomatitis virus G glycoproteins (VSVG)), 50 µl of 2.5 mol CaCl₂, and ddH₂O up to 500 µl, and the solution B includes 500 µl of 2× HEPES salt buffer; after the solution A is prepared, standing it for 3 minutes, then adding the solution A to the solution B while bubbling in a reagent tube with an electronic pipette to mix the solution A and the solution B evenly until the solution in the reagent tube becomes white and turbid, thereby preparing the transfection reagent; and adding 1 ml of the transfection reagent to the culture dish containing the 293T cells, gently shaking to mix evenly, and placing the culture dish in an incubator at 37°C for transfection;
12 to 16 hours post the transfection, performing a medium change by discarding culture supernatant and adding 15 ml of Dulbecco's modified Eagle's medium (DMEM) medium containing 10% fetal bovine serum (FBS);
48 hours post the medium change, filtering the solution through a 0.45-µm filter, collecting 48-hour culture supernatant, making a supplement with 10 ml of DMEM medium containing 10% FBS, and storing it at 4°C;
24 hours post the storage, filtering the solution through a 0.45-µm filter and collecting72-hour culture supernatant; and
concentrating both the 48-hour culture supernatant and the 72-hour culture supernatant by 50-fold with a concentration pump, then filtering the solution through a 0.22-µm filter, collecting the filtered virus concentrate and dispensing it to a 1.5-mL Eppendorf (EP) tube, and then storing it at -80°C to obtain the viral particles.

In a preferred embodiment of the present application, in the preparation method of the aforementioned immune cell, the step of preparing the immune cell with the viral particles includes:
retrieving the viral particles and reobtaining a virus concentrate;
collecting human anticoagulant peripheral blood, diluting it with an equal volume of phosphate buffer saline (PBS), and separating the diluted human anticoagulant peripheral blood with a human lymphocyte separation medium to collect peripheral blood mononuclear cells (PBMCs);
adjusting a concentration of T cells in the PBMCs to 1×10⁷/ml, adding anti-CD3 and anti-CD28 magnetic beads to sort the T cells, and activating them to bring the T cells into an activated state;
24 hours post the activation of the T cells, inoculating the T cells into a 24-well plate at a density of 5×10⁵ cells/mL, and adding the virus concentrate and 1 ml of an infection culture medium for virus infection;
6 hours post the infection, making a supplement with 1 ml of a fresh infection culture medium;
continuing the infection for 48 hours, discarding culture supernatant, and replacing it with a fresh infection culture medium;
continuing the infection to expand the T cells for 6 to 8 days to obtain the immune cell.

The beneficial effects of the embodiments of the present application are as follows:
The immune cell of the present application, in addition to exerting the anti-tumor efficacy of a common immune cell, is capable of expressing and secreting the fusion protein including the anti-PD-1 scFv and the IL-21. The secreted fusion protein can block the PD-1 inhibitory signals on CAR-T cells, while simultaneously targeting the IL-21 to the immune cell to promote the proliferation of the immune cell and differentiation into a memory cell, which can significantly enhance the anti-tumor efficacy of the immune cell and improve the therapeutic efficacy against tumors.

Since cytokines act on numerous types of target cells, their systemic administration results in substantial adverse effects, greatly limiting their use in disease treatment. The fusion protein secreted by the PD-1Ab21-CAR-T cell (PD-1Ab21-TCR-T cell, PD-1Ab21-CAR-NK cell, and PD-1Ab21-TIL cell) can target PD-1⁺ anti-tumor T cells in vivo, reducing off-target effects on other cells and greatly mitigating the systemic adverse effects of the cytokine IL-21.

### Brief Description of the Drawings

In order to describe the technical solutions of the embodiments of the present application more clearly, the drawings used in the embodiments of the present application will be briefly introduced below. It should be understood that the drawings described below only illustrate some embodiments of the present application, and therefore should not be regarded as restrictions on the scope. For those of ordinary skill in the art, other relevant drawings can also be obtained according to these drawings without creative effort.
FIG. 1 shows a schematic diagram illustrating a construction structure of an expression sequence of the present application;
FIG. 1a and FIG. 1b show schematic diagrams illustrating construction structures of a fusion protein of the present application; FIG. 1c shows a schematic diagram of a construction structure of an expression plasmid targeting a specific antigen but not including the fusion protein of the present application; FIG. 1d and FIG. 1e show schematic diagrams of construction structures of an expression plasmid targeting a specific antigen and including the fusion protein of the present application;
FIG. 2 shows a schematic diagram illustrating preparation of a PD-1Ab21-CD19CAR-T cell of the present application and functional characterization of a secreted PD-1Ab21 fusion protein;
FIG. 2a shows a schematic diagram illustrating a proportion of CAR⁺ cells in the prepared PD-1Ab21-CD19CAR-T cells;
FIG. 2b shows a schematic diagram illustrating binding of the PD-1Ab21 fusion protein in culture supernatant of the PD-1Ab21-CD19CAR-T cell to a CHO cell expressing PD-1 (CHO-PD-1), where 2×10⁵ of CHO-PD-1 cells were incubated with CAR-T cell culture supernatant (concentrated by 30-fold with an ultrafiltration tube; 1 ml) at 4°C for 30 minutes, and 3 µg of an anti-PD-1Ab-scFv was incubated for 30 minutes in parallel as a control; and proteins bound on the CHO-PD-1 cells were detected via a PE-labeled anti-His antibody;
FIG. 2c shows a schematic diagram illustrating a blocking effect of the PD-1Ab21 fusion protein in culture supernatant of the PD-1Ab21-CD19CAR-T cell on PD-1 molecules on a surface of the CHO-PD1 cell, where 2×10⁵ of CHO-PD-1 cells were incubated with CAR-T culture supernatant (concentrated by 30-fold with an ultrafiltration tube; 1 ml) at 4°C for 30 minutes; and expression of PD-1 on CHO-PD-1 cells was detected via an APC-labeled anti-hPD-1 antibody (the detection result is negative if a blocking effect is present);
FIG. 2d shows a schematic diagram illustrating an activation effect of a PD-1Ab21 fusion protein in culture supernatant of the PD-1Ab21-CD19CAR-T cell on IL-21R, where Baf3 cells were inoculated into a 24-well plate at a density of 5×10⁵ per well, then incubated with 0.3 µM of IL-21 (positive control) or CAR-T supernatant for 30 minutes, and subjected to intracellular staining with an anti-pSTAT3 antibody; and phosphorylation level of STAT3 in cells was detected by flow cytometry;
FIG. 3 shows a schematic diagram comparing proliferation abilities and differentiation phenotypes of a PD-1Ab21-CD19CAR-T cell and a CD19CAR-T cell of the present application cultured in vitro;
FIG. 3a shows cell proliferation curves of the prepared CD19CAR-T and PD-1Ab21-CD19CAR-T cells which are cultured in vitro for 11 days with 100 U/ml (left panel) or 10 U/ml (right panel) of IL-2, respectively, where cells counts were performed daily to determine proliferation fold of the CD19CAR-T cells and the PD-1Ab21-CD19CAR-T cells, based on which the proliferation curves were plotted.
FIG. 3b shows an analysis of phenotypes (CD45RA and CCR7) of CAR-T cells collected on day 14 of culture for both the CD19CAR-T cells and the PD-1Ab21-CD19CAR-T cells by flow cytometry;
FIG. 3c shows an analysis of a proportion of T_{SCM} (CD45RA⁺CCR7⁺) cells in CD4⁺ CAR-T cells and CD8⁺ CAR-T cells by flow cytometry, where the CD19CAR-T cells and the PD-1Ab21-CD19CAR-T cells were cultured in presence of 10 U/ml of IL-2, the cultured CAR-T cells were sampled and stained at indicated time points.
FIG. 3d shows an analysis of proportions of CD45RA⁺, CCR7⁺, CD62L⁺, CD45RO⁺, CD28⁺ cells in CD4⁺ and CD8⁺ CAR-T cells by flow cytometry, where the CAR-T cells cultured for 14 days were sampled and stained;
FIG. 4 shows a schematic diagram illustrating in vitro killing function detection for PD-1Ab21-CD19CAR-T cells of the present application;
FIG. 4a shows an assay of killing capability of the CAR-T cells by flow cytometry, where the CD19CAR-T cells and the PD-1Ab21-CD19CAR-T cells were cultured in vitro with 100 U/ml of IL-2 until day 5, then a predetermined number of CAR-T cells were mixed and co-cultured with Daudi-PDL1 cells at an E (CAR-T):M (Daudi-PDL1) ratio as shown in the figure respectively for 24 hours;
FIG. 4b shows an evaluation of sustained killing capability of the CD19CAR-T cells and the PD-1Ab21-CD19CAR-T cells, where the CAR-T cells cultured in vitro with 100 U/ml of IL-2 until day 5 were co-cultured with Daudi-PDL1 cells at an indicated E (CAR-T):M (Daudi-PDL1) ratio for 8 days;
FIG. 4c shows the changes in total T-cell numbers and CD8/CD4 ratios during the sustained killing assay of the CAR-T cells, where in the killing experiment shown in the above FIG. 4b, cells were sampled at various time points for counting, and analyzed by flow cytometry for the proportions of CD3⁺, CD4⁺, and CD8⁺ T cells to calculate total amount of CD3⁺ T cells and CD8/CD4 ratio, and their changes over time were plotted;
FIG. 5 shows a schematic diagram illustrating in vitro proliferation and cytokine secretion abilities of PD-1Ab21-CD19CAR-T cells of the present application;
FIG. 5a shows detection of Ki-67 expression in the CD19CAR-T cells and the PD-1Ab21-CD19CAR-T cells, where CAR-T cells cultured in vitro with 100 U/ml of IL-2 until day 5 were co-cultured with Daudi-PDL1 cells at an E (CAR-T):M (Daudi-PD-L1) ratio of 1:5 for 48 hours;
FIG. 5b shows detection of secretion and expression of IL-2 and IFN-γ by the CD19CAR-T cells and the PD-1Ab21-CD19CAR-T cells under a culture condition of FIG. 5a by flow cytometry;
FIG. 5c shows detection of levels of IL-2 and IFN-γ secretion in culture supernatant of the CD19CAR-T cells and the PD-1Ab21-CD19CAR-T cells under a condition of FIG. 5a by ELISA(C);
FIG. 6 shows a schematic diagram of exhaustion phenotype detection for a PD-1Ab21-CD19CAR-T cell of the present application;
FIG. 6a shows detection of proportions of T cells and tumor cells for the CD19CAR-T cells and the PD-1Ab21-CD19CAR-T cells, where CAR-T cells cultured in vitro with 100 U/ml of IL-2 until day 5 were co-cultured with Daudi-PDL1 cells at an E (CAR-T):M (Daudi-PDL1) ratio of 1:5 for 8 days;
FIG. 6b shows detection of numbers of T cells and tumor cells for the CD19CAR-T cells and the PD-1Ab21-CD19CAR-T cells, where CAR-T cell cultured in vitro with 100 U/ml of IL-2 until day 5 were co-cultured with Daudi-PDL1 cells at an E (CAR-T):M (Daudi-PDL1) ratio of 1:5 for 8 days;
FIG. 6c shows expressions of exhaustion markers PD-1, TIGIT, TOX, LAG3 on CD8 T cells for the CD19CAR-T cells and the PD-1Ab21-CD19CAR-T cells, where CAR-T cells cultured in vitro with 100 U/ml of IL-2 until day 5 were co-cultured with Daudi-PDL1 cells at an E (CAR-T):M (Daudi-PDL1) ratio of 1:5 for 8 days;
FIG. 7 shows a schematic diagram illustrating that PD-1Ab21-CD19CAR-T cells in immune cells of the present application exhibit significant anti-tumor efficacy in a humanized mouse model of B-cell leukemia;
FIG. 7a shows an inoculation of Raji-luc cells in NSG mice through tail vein injection, and reinfusion of 5×10⁶ CD19CAR-T and PD-1Ab21-CD19CAR-T cells of CAR⁺ 7 days after inoculation;
FIG. 7b shows tumor progression monitored by small animal in vivo imaging;
FIG. 8 shows a schematic diagram illustrating that PD-1Ab21-CD19CAR-T cells in immune cells of the present application exhibit significant anti-tumor efficacy in a humanized mouse model of lymphoma;
FIG. 8a shows an inoculation of Daudi-hPD-L1 cells in NSG mice through subcutaneous injection, and reinfusion of 2×10⁶ CD19CAR-T and PD-1Ab21-CD19CAR-T cells of CAR⁺ 19 days after inoculation;
FIG. 8b shows survival curves of NSG mice after the subcutaneous inoculation of Daudi-hPD-L1 cells, where a PD-1Ab21-CD19CAR-T cell treatment group exhibits a higher survival rate compared with a CD19CAR-T cell treatment group;
FIG. 8c shows tumor growth curves, with tumor length (a) and width (b) measured, and a tumor volume calculated as (ab²/2);
FIG. 8d shows an analysis of percentage of reinfused CD3⁺CD45⁺CAR-T cells in peripheral blood of tumor-bearing NSG mice by flow cytometry;
FIG. 9 shows a schematic diagram illustrating that PD-1Ab21 of the present application enhances proliferation and stem-like differentiation of CAR-T cells in an immune cell, thereby mediating stronger anti-tumor efficacy;
FIG. 9a shows inoculation of a Daudi-hPD-L1 cell in NSG mice through subcutaneous injection, and reinfusion of 5×10⁶ CD19CAR-T and PD-1Ab21-CD19CAR-T cells of CAR⁺ 33 days after the inoculation, where on day 12 post treatment, peripheral blood, spleen, and tumors were collected from mice for flow cytometric analysis;
FIG. 9b shows proportions of CD3⁺CD8⁺ CAR-T cells in the peripheral blood, spleen, and tumor tissues from NSG mice;
FIG. 9c shows detection of Ki-67 expression of CD3⁺CD8⁺ CAR-T cells in the spleen and tumor tissues by flow cytometry, and statistical analysis of a positive rate of Ki67 in the CD3⁺CD8⁺ CAR-T cells;
FIG. 9d shows detection of phenotypes of the CD3⁺CD8⁺ CAR-T cells at tumor site by flow cytometry, and statistical analysis of proportions of stem-like memory CD8⁺ T cells in CD45RA⁺CD62L⁺ and central memory CD8⁺ T cells in CD45RA⁻CD62L⁺;
FIG. 10 shows a schematic diagram illustrating that PD-1Ab21-BCMACAR-T cells in immune cells of the present application exhibit significant anti-tumor efficacy in a humanized mouse model of myeloma;
FIG. 10a shows inoculation of a U266-luc cell in NSG mice through tail vein injection, and reinfusion of 2×10⁶ BCMACAR-T and PD-1Ab21-BCMACAR-T cells of CAR⁺ 24 days after the inoculation;
FIG. 10b shows tumor progression monitored by small animal in vivo imaging;
FIG. 11 shows a schematic diagram illustrating that PD-1Ab21-Her2CAR-T cells in immune cells of the present application exhibit significant anti-tumor efficacy in a humanized mouse model of breast cancer;
FIG. 11a shows inoculation of a BT474-PD-L1 cell in NSG mice through subcutaneous injection, and reinfusion of 3×10⁶ Her2CAR-T and Her2CAR-T-hPD-1Ab21 cells of CAR⁺ 34 days after the inoculation;
FIG. 11b shows survival curves of tumor-bearing mice, where a PD-1Ab21-Her2CAR-T cell treatment group exhibits a higher survival rate compared with a Her2CAR-T cell treatment group; and
FIG. 11c shows tumor growth curves, with tumor length (a) and width (b) measured, and a tumor volume calculated as (ab²/2).

### Detailed Description of Embodiments

In order to make the objectives, technical solutions, and advantages of the embodiments of the present application clearer, the technical solutions in the embodiments of the present application will be described clearly and completely below in conjunction with the accompanying drawings in the embodiments of the present invention. Obviously, the described embodiments are only part of the embodiments of the present application rather than all of them. Generally, the components of the embodiments of the present application described and illustrated in the accompanying drawings herein can be arranged and designed in various different configurations.

In particular, it is well known that due to the degeneracy of nucleotide codons, each amino acid sequence can correspond to multiple nucleotide coding sequences. Therefore, the coding nucleotide sequences of the amino acid sequence given in the present application are merely exemplary. For each amino acid sequence, all nucleotide sequences capable of encoding the amino acid sequence are functional equivalents and fall within the scope of the present application.

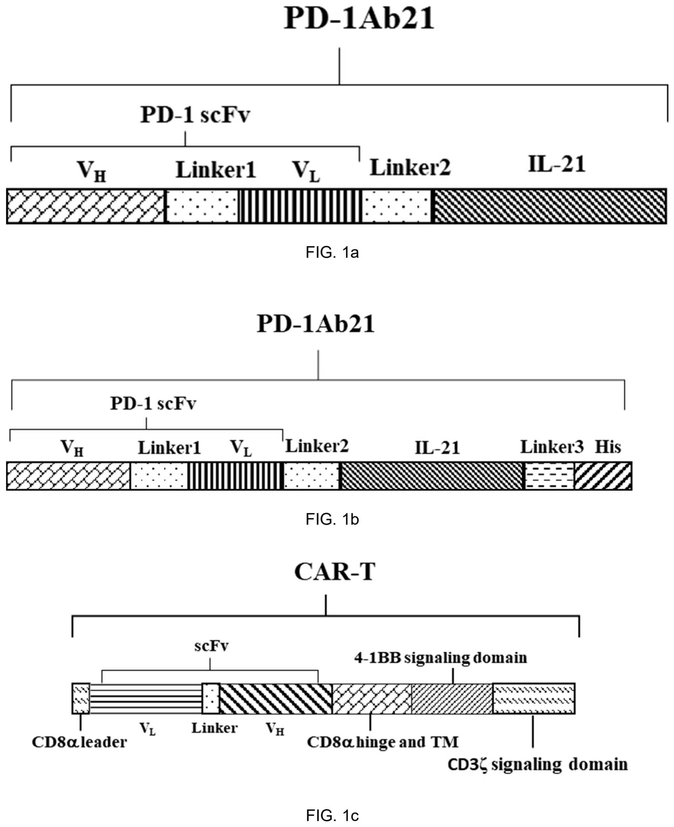

The present application provides a fusion protein PD-1Ab21 including an anti-PD-1 scFv and IL-21, and a specific structure of the fusion protein is shown in FIG. 1a and FIG. 1b.

In a preferred embodiment of the present application, the anti-PD-1 scFv includes a heavy chain variable region and a light chain variable region, where the heavy chain variable region includes HCDR1, HCDR2, and HCDR3, which include or consist of amino acid sequences of SEQ ID No.69, SEQ ID No.70, and SEQ ID No.71, respectively; the light chain variable region includes LCDR1, LCDR2, and LCDR3, which include or consist of amino acid sequences of SEQ ID No.72, SEQ ID No.73, and SEQ ID No.74, respectively; and the IL-21 includes or consists of an amino acid sequence of SEQ ID No.16.

In a preferred embodiment of the present application, the heavy chain variable region of the anti-PD-1 scFv can include or consist of an amino acid sequence of SEQ ID No. 8, and the light chain variable region can include or consist of an amino acid sequence of SEQ ID No.12.

In a preferred embodiment of the present application, the heavy chain variable region and the light chain variable region of the anti-PD-1 scFv can be linked together via linker1, which includes or consists of an amino acid sequence of SEQ ID No. 10.

In a preferred embodiment of the present application, the IL-21 can include or consist of an amino acid sequence of SEQ ID No. 16.

In a preferred embodiment of the present application, the anti-PD-1 scFv and the IL-21 are linked together via linker2 to form the fusion protein PD-1Ab21. Exemplarily, the linker2 can include or consist of an amino acid sequence of SEQ ID No.14.

In an alternative embodiment, a C-terminus of the fusion protein further includes a His tag. In a specific embodiment, the His tag in the fusion protein is linked to the C-terminus via linker 3. Exemplarily, the linker3 can include or consist of an amino acid sequence of SEQ ID. No.18.

In a specific embodiment, the fusion protein PD-1Ab21 includes or consists of an amino acid sequence of SEQ ID No. 2 or SEQ ID.No.4. Exemplarily, coding nucleotide sequences of SEQ ID No.2 and SEQ ID No.4 are shown in SEQ ID No.1 and SEQ ID No.3, respectively. The present application also provides an immune cell which is capable of expressing and secreting the aforementioned fusion protein.

In a preferred embodiment of the present application, the aforementioned immune cell specifically recognizes at least one of a hematologic malignancy, a lung cancer, a breast cancer, a liver cancer, a pancreatic cancer, a renal cancer, a prostate cancer, an ovarian cancer, a gastrointestinal tumor, a brain tumor, a neuroendocrine tumor, a bone tumor, and a soft tissue tumor.

In a preferred embodiment of the present application, the aforementioned immune cell is selected from at least one of CAR-T cells, TCR-T cells, CAR-NK cells, and TIL cells.

In a preferred embodiment of the present application, the aforementioned immune cell not only exerts its own anti-tumor efficacy, but also enhances inherent anti-tumor immune responses in a body.

The present application provides a viral vector which is capable of expressing the fusion protein PD-1Ab21.

In a preferred embodiment of the present application, after the viral expression vector infects a cell, it causes the cell to secrete the fusion protein PD-1Ab21.

The present application provides an immune cell expression vector including an expression plasmid targeting a specific antigen, a P2A sequence fragment, and a coding sequence fragment of the fusion protein PD-1Ab21.

Preferably, the immune cell expression vector further includes a His tag.

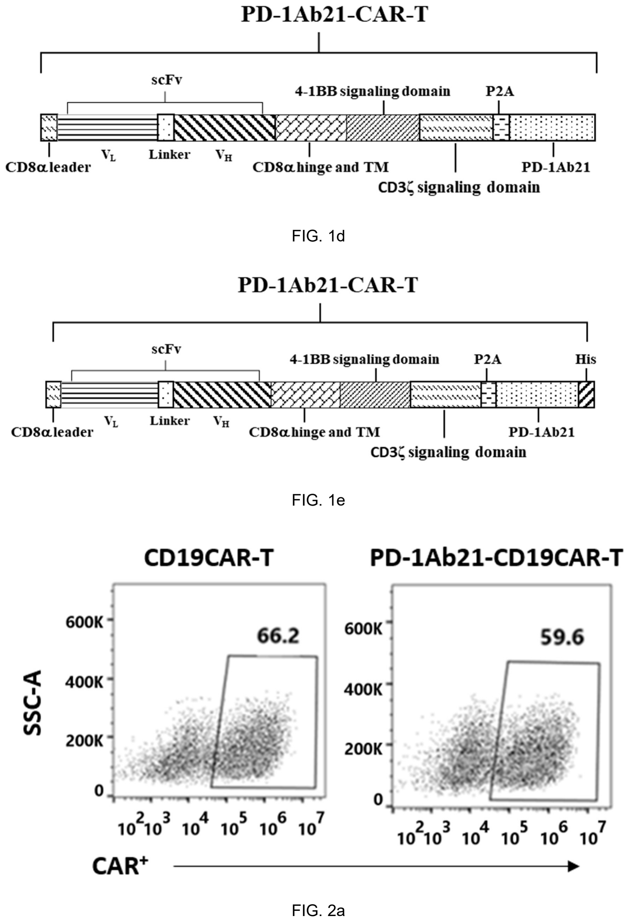

The structure of the expression plasmid targeting a specific antigen is shown in FIG. 1d and FIG. 1e, which includes an extracellular domain, a transmembrane domain, and an intracellular domain.

The extracellular domain includes a CD8α signal peptide fragment (the region indicated as "CD8α leader" in FIG. 1d and FIG. 1e), a specific antibody fragment (the region indicated as "scFv" in FIG. 1d and FIG. 1e, including a heavy chain variable region VH, a light chain variable region VL, and Linker), and a CD8α hinge region fragment (the region indicated as "CD8α hinge" in FIG. 1d and FIG. 1e).

The transmembrane domain includes a CD8α transmembrane region fragment (the region indicated as "CD8α TM" in FIG. 1d and FIG. 1e).

The intracellular domain includes a 4-1BB signaling domain fragment (the region indicated as "4-1BB signaling domain" in FIG. 1d and FIG. 1e) and a CD3ζ signaling domain fragment (the region indicated as "CD3ζ signaling domain" in FIG. 1d and FIG. 1e).

In a preferred embodiment of the present application, an amino acid sequence of the fusion protein PD-1Ab21 in the aforementioned immune cell expression vectors is shown in SEQ ID No.2 or SEQ ID No.4, and a cDNA sequence of the coding sequence fragment of the fusion protein PD-1Ab21 is shown in SEQ ID No.1 or SEQ ID No.3, respectively.

In a preferred embodiment of the present application, the specific antibody fragment in the aforementioned immune cell expression vector includes a specific scFv targeting CD19, and/or a specific scFv targeting BCMA, and/or a specific antibody fragment targeting a Her2/neu tumor antigen, and/or a specific antibody fragment targeting other tumor antigens.

In a preferred embodiment of the present application, a nucleotide sequence and an amino acid sequence of a light chain variable region of the specific scFv targeting CD19 are shown in SEQ ID No.25 and SEQ ID No.26, respectively, and a nucleotide sequence and an amino acid sequence of a heavy chain variable region are shown in SEQ ID No.29 and SEQ ID No.30, respectively.

In a specific embodiment of the present application, the light chain variable region and the heavy chain variable region of the specific scFv targeting CD19 are linked together via linker4. Exemplarily, a nucleotide sequence and an amino acid sequence of the linker4 can be shown in SEQ ID No.27 and SEQ ID No.28, respectively.

In an exemplary embodiment of the present application, a nucleotide sequence and an amino acid sequence of the specific scFv targeting CD19 are shown in SEQ ID No.31 and SEQ ID No.32, respectively.

In a preferred embodiment of the present application, a nucleotide sequence and an amino acid sequence of a light chain variable region of the specific scFv targeting BCMA are shown in SEQ ID No.47 and SEQ ID No.48, respectively, and a nucleotide sequence and an amino acid sequence of a heavy chain variable region are shown in SEQ ID No.51 and SEQ ID No.52, respectively.

In a specific embodiment of the present application, the light chain variable region and the heavy chain variable region of the specific scFv targeting BCMA are linked together via linker5. Exemplarily, a nucleotide sequence and an amino acid sequence of the linker5 can be shown in SEQ ID No.49 and SEQ ID No.50, respectively.

In an exemplary embodiment of the present application, a nucleotide sequence and an amino acid sequence of the specific scFv targeting BCMA are shown in SEQ ID. 45 and SEQ ID No.46, respectively.

In a preferred embodiment of the present application, a nucleotide sequence and an amino acid sequence of a light chain variable region of the specific scFv targeting Her2 are shown in SEQ ID No.59 and SEQ ID No.60, respectively, and a nucleotide sequence and an amino acid sequence of a heavy chain variable region are shown in SEQ ID No.63 and SEQ ID No.64, respectively.

In a specific embodiment of the present application, the light chain variable region and the heavy chain variable region of the specific scFv targeting Her2 are linked together via linker6. Exemplarily, a nucleotide sequence and an amino acid sequence of the linker6 can be shown in SEQ ID No.61 and SEQ ID No.62, respectively.

In an exemplary embodiment of the present application, a nucleotide sequence and an amino acid sequence of the specific scFv targeting Her2 are shown in SEQ ID No.57 and SEQ ID No.58, respectively.

The present application provides a use of an immune cell in treating a B-cell leukemia, a B-cell lymphoma, a myeloma, a Her2/neu⁺ breast cancer, and an advanced B-cell malignancy, where the immune cell is the immune cell as described above.

The present application provides a treatment method of a B-cell leukemia, a B-cell lymphoma, a myeloma, a Her2/neu⁺ breast cancer, and an advanced B-cell malignancy, where a therapeutically effective amount of the aforementioned immune cell is administered to a subject.

The present application provides a preparation method of an immune cell, including steps of:
constructing a coding gene of the fusion protein PD-1Ab21;
constructing an immune cell expression vector expressing the fusion protein PD-1Ab21;
performing virus packaging on the immune cell expression vector to obtain viral particles; and
preparing the immune cell with the viral particles.

In a preferred embodiment of the present application, in the preparation method of the aforementioned immune cell, the step of constructing a coding gene of the fusion protein PD-1Ab21 includes:
selecting a humanized PD-1 antibody and linking a heavy chain variable region with a light chain variable region together via Linker1 to obtain an anti-PD-1 scFv targeting specific cells; and
linking the anti-PD-1 scFv targeting specific cells with a cDNA sequence of a fusion protein including an IL-21 cytokine via the linker Linker2 and performing DNA fragment synthesis to obtain the coding gene of the fusion protein PD-1Ab21.

In a preferred embodiment of the present application, in the preparation method of the aforementioned immune cell, the step of constructing an immune cell expression vector expressing the fusion protein PD-1Ab21 includes:
constructing an expression plasmid targeting a specific antigen with a lentiviral vector pCDH, where the expression plasmid targeting a specific antigen includes an extracellular domain, a transmembrane domain, and an intracellular domain;
the extracellular domain includes a CD8α signal peptide fragment (the region indicated as "CD8α leader" in FIG. 1d and FIG. 1e), a specific antibody fragment (the region indicated as "scFv" in FIG. 1d and FIG. 1e, including a heavy chain variable region VH, a light chain variable region VL, and Linker), and a CD8α hinge region fragment (the region indicated as "CD8α hinge" in FIG. 1d and FIG. 1e);
the specific antibody fragment includes a specific scFv targeting CD19, and/or a specific scFv targeting BCMA, and/or a specific antibody fragment targeting a Her2/neu tumor antigen, and/or a specific antibody fragment targeting other tumor antigens;
the transmembrane domain includes a CD8α transmembrane region fragment (the region indicated as "CD8α TM" in FIG. 1d and FIG. 1e); and
the intracellular domain includes a 4-1BB signaling domain fragment (the region indicated as "4-1BB signaling domain" in FIG. 1d and FIG. 1e) and a CD3ζ signaling domain fragment (the region indicated as "CD3ζ signaling domain" in FIG. 1d and FIG. 1e); linking a coding sequence fragment of the fusion protein PD-1Ab21 to downstream of a coding fragment of the expression plasmid targeting a specific antigen via a P2A sequence fragment; and
optionally, attaching a His tag to a C-terminus of an entire sequence to obtain the immune cell expression vector expressing the fusion protein PD-1Ab21.

Exemplarily, nucleotide sequences and amino acid sequences of the specific scFv targeting CD19, the specific scFv targeting BCMA, and the specific antibody fragment targeting a Her2/neu tumor antigen are as described above. In a preferred embodiment of the present application, in the preparation method of the aforementioned immune cell, the step of performing virus packaging on the immune cell expression vector to obtain viral particles includes:
performing the virus packaging with a calcium phosphate transfection method;
16 to 20 hours before transfection, selecting 293T cells for subculture and inoculating 4×10⁶ 293T cells into each cell culture dish;
20 to 24 hours post the inoculation, observing the subculture of the 293T cells and performing the transfection when a coverage rate of the 293T cells reaches 50%; where
the transfection method includes: preparing a transfection reagent including solution A and solution B, where the solution A includes 35 µg of plasmids (15 µg of CAR expression vector plasmids, 7.5 µg of a helper plasmid RRE, 6 µg of REV, and 6 µg of VSVG), 50 µL of 2.5 mol CaCl2, and ddH₂O up to 500 µl, and the solution B includes 500 µl of 2× HEPES salt buffer; after the solution A is prepared, standing it for 3 minutes, then adding the solution A to solution B while bubbling in a reagent tube with an electronic pipette to mix the solution A and the solution B evenly until the solution in the reagent tube becomes white and turbid, thereby preparing the transfection reagent; and adding 1 ml of the transfection reagent to the culture dish containing the 293T cells, gently shaking to mix evenly, and placing the culture dish in an incubator at 37°C for transfection;
12 to 16 hours post the transfection, performing a medium change by discarding a culture supernatant and adding 15 ml of DMEM medium containing 10% FBS;
48 hours post the medium change, filtering the solution through a 0.45-µm filter, collecting 48-hour culture supernatant, making a supplement with 10 ml of DMEM medium containing 10% FBS, and storing it at 4°C;
24 hours post the storage, filtering the solution through a 0.45-µm filter and collecting 72-hour culture supernatant; and
concentrating both the 48-hour culture supernatant and the 72-hour culture supernatant by 50-fold with a concentration pump, then filtering the solution through a 0.22-µm filter, collecting the filtered virus concentrate and dispensing it into a 1.5 mL EP tube, and then storing it at -80°C to obtain the viral particles.

In a preferred embodiment of the present application, in the preparation method of the aforementioned immune cell, the step of preparing the immune cell with the viral particles includes:
retrieving the viral particles and reobtaining the virus concentrate;
collecting human anticoagulant peripheral blood, diluting it with an equal volume of PBS, and separating the diluted human anticoagulant peripheral blood with a human lymphocyte separation medium to collect PBMCs;
adjusting a concentration of T cells in the PBMCs to 1×10⁷/ml, adding anti-CD3 and anti-CD28 magnetic beads to sort the T cells, and activating them to bring the T cells into an activated state;
24 hours post the activation of the T cells, inoculating the T cells into a 24-well plate at a density of 5×10⁵ cells/mL, and adding the virus concentrate (MOI=5) and 1 ml of an infection culture medium (X-VIVO^{™}15 Medium, containing 200U/mL of IL-2 and 8 µg/mL of Polybrene) for virus infection;
6 hours post the infection, making a supplement with 1 ml of a fresh infection culture medium;
continuing the infection for 48 hours, discarding culture supernatant, and replacing it with a fresh infection culture medium (X-VIVO^{™}15 Medium, containing 100U/mL of IL-2);
continuing the infection to expand the T cells for 6 to 8 days to obtain the immune cell.

The present application provides a method for detecting and functionally characterizing a fusion protein PD-1Ab21 in culture supernatant of a PD-1Ab21-CD19CAR-T cell:
Prepared PD-1Ab21-CD19CAR-T cells and conventional CD19CAR-T cells are cultured separately in a CAR-T cell culture medium for 48 hours, and then supernatant is collected and a concentration of a fusion protein PD-1Ab21 in the supernatant is detected by ELISA. To evaluate the ability of the fusion protein PD-1Ab21 in the supernatant to bind the PD-1 molecule expressed on the cell surface, the CHO (PD-1⁺CHO) cell strains stably expressing PD-1 is resuspended in FACS buffer, and incubated with a certain amount of CAR-T cell culture supernatant or recombinantly expressed humanized anti-PD-1 scFv protein at 4°C for 20 minutes, and then the supernatant is washed off by centrifugation, then the CHO cells are resuspended in FACS buffer, and incubated with a fluorescently-labeled anti-His antibody (the fusion protein PD-1Ab21 carrying the His tag) at 4°C for 20 minutes, the anti-His antibody is washed off by centrifugation, and the fusion protein PD-1Ab21 bound to the surface of CHO cells is detected by flow cytometry. The results show that the ability of the fusion protein PD-1Ab21 in the CAR-T cell culture supernatant to bind the PD-1 molecule on the cell surface is comparable to that of the humanized anti-PD-1 scFv. When the PD-1⁺CHO cells expressing PD-1 are incubated with PD-1Ab21-CD19CAR-T or conventional CD19CAR-T cell culture supernatant for 20 minutes and subsequently incubated with fluorescently-labeled anti-PD-1 antibody at 4°C for 20 minutes, the anti-PD-1 antibody is washed off by centrifugation, and the PD-1 antibody bound to the surface of CHO cells is detected by flow cytometry and results show that: the PD-1Ab21-CD19CAR-T cell culture supernatant can block the binding of anti-PD-1 antibody to CHO cells, while the conventional CD19CAR-T cell culture supernatant does not exhibit such a blocking effect.

To evaluate the biological activity of IL-21 in the fusion protein PD-1Ab21 present in the culture supernatant, the Baf3 cells expressing IL-21R were inoculated into a 24-well plate at a density of 5×10⁵ cells/well, an appropriate amount of a PD-1Ab21-CD19CAR-T cell culture supernatant or 0.3 uM of a recombinant IL-21 (positive control) was added for co-culture for 30 minutes, and the intracellular flow staining was then performed to measure the phosphorylation degree of STAT3. The results show that the CAR-T culture supernatant exhibits the same biologic activity for stimulating the activation of IL-21R as recombinant IL-21. These findings indicate that the fusion protein secreted by the CAR-T cells not only possesses both binding and blocking functions of PD-1 antibody, but also exerts the normal biological activity of IL-21.

The method for detecting the concentration of fusion protein PD-1Ab21 in CAR-T culture supernatant by ELISA is as follows: the plate is coated with PD-1 protein at 0.1 µg per well at 4°C overnight; the plate is washed three times; 100 µl of 10% FBS is added per well for blocking 30-60 minutes; the plate is washed three times, the concentration of standard samples is multiple diluted in a gradient from 200 ng/ml, and 50 µl is added per well, the samples are tested at stock solution/10×/100×/1000× dilutions and incubated at room temperature for 2 hours; the plate is washed three times, anti-IL-21-HRP antibody (600×) is added at 50 µl per well, and incubated at room temperature for 1 hour in the dark; the plate is washed five times, and 50 µl of TMB is added per well for color development, the color development time is determined based on the degree of color development of the standard curve, which is generally about 2-3 minutes depending on the color development effect of standard curve, 50ul of 1M H₂SO₄ is added per well to terminate the reaction, and the reading at 460 nm OD is analyzed.

The present application further provides a recognition method of in-vitro proliferation and phenotype for PD-1Ab21-CD19CAR-T cells.

To investigate the effect of expressing and secreting of PD-1Ab21 on in vitro proliferation and differentiation of CAR-T cells, the prepared PD-1Ab21-CD19CAR-T and conventional CD19CAR-T cells were cultured for 11 days in culture medium containing 100 U/ml or 10 U/ml of IL-2, the number of CD19CAR-T cells were counted daily, and the fold proliferation of PD1Ab21-CD19CAR-T cells was calculated. The results show that when cultured in culture medium containing 100 U/ml of IL-2, the proliferations of two CAR-T cells have no difference; however, when cultured in culture medium containing 10 U/ml of IL-2, the number of CD19CAR-T cell numbers shows no significantly increase, whereas the PD-1Ab21-CD19CAR-T cells have significant expansion. The phenotypes of the cultured CAR-T cells were analyzed by flow cytometry, the results show that under the culture condition of 100 U/ml of IL-2, the proportion of stem-like memory T cells (T_{SCM}, CD45RA⁺CCR7⁺) in the PD-1Ab21-secreting CAR-T cells is higher than that of conventional CD19CAR-T cells, and the difference is even more significant under the culture condition of 10 U/ml of IL-2, and it is found that across all tested time points, the proportion of stem-like memory T cells in the PD-1Ab21-secreting CAR-T cells is significantly higher than that of conventional CD19CAR-T cells. These results demonstrate that the PD-1Ab21-CD19CAR-T cells tend to differentiate into stem-like memory T cells.

To evaluate the proliferative ability of the CAR-T cells after being stimulated by tumor antigens, the prepared PD-1Ab21-CD19CAR-T and CD19CAR-T cells were cultured with the target Daudi-PDL1 cells at a ratio of 1:5 in 96-well cell culture plates under the culture condition of 100 U/ml of IL-2 for 2 days, and the Ki-67 expression on the surface of CAR-T cells was detected by flow cytometry. The results show that the Ki-67 expression of PD-1Ab21-CD19CAR-T cells is significantly higher than that of CD19CAR-T cells, indicating that the proliferative ability of PD-1Ab21-CD19CAR-T cells is significantly higher than that of CD19CAR-T cells.

The present application provides an in vitro cytotoxicity assay and cytokine production assay for CAR-T cells, including:
cultivating and amplifying the CD19CAR-T and PD-1Ab21-CD19CAR-T cells in culture medium containing 100 U/ml of IL-2 for 5 days, then co-cultivating with Daudi-PD-L1 target cells at different effector-to-target ratios (1:1, 2:1, 5:1, 10:1) in a 96-well plate for 24 hours, detecting the killing activity of CAR-T cells on target cells by flow cytometry. The results show that there is no significant difference in killing activity between CD19CAR-T and PD-1Ab21-CD19CAR-T cells against CD19⁺ tumor cells. However, when co-cultivated with tumor cells at a low effector-to-target ratio of 1:5 for 8 days, the PD-1Ab21-CD19CAR-T cells exhibit significantly higher sustained killing capability against tumor cells compared with CD19CAR-T cells, and moreover, the proliferation of PD-1Ab21-CD19CAR-T cells is also significantly higher than that of CD19CAR-T cells. The phenotype of CAR-T cells was detected by flow cytometry on day 8, and the results show that the expression of immune checkpoint molecules (PD-1, TOX, TIGIT) on PD-1Ab21-CD19CAR-T cells is significantly lower than that of CD19CAR-T cells.

To detect the cytokine producing ability of CAR-T cells, the prepared PD-1Ab21-CD19CAR-T and CD19CAR-T cells were separately co-cultivated with target cell Daudi-PD-L1 cells at a ratio of 1:5 in the culture medium containing 100 U/ml of IL-2 in 24-well cell culture plate for 2 days, then the cells and supernatants were collected, and intracellularly stained, and the cells expressing cytokine were detected by flow cytometry. The results show that the proportions of IL-2 positive and IFN-γ positive cells in PD-1Ab21-CD19CAR-T cells are significantly higher than that of CD19CAR-T cells. At the same time, the cytokines in culture supernatant were detected with ELISA kit. The results show that the concentration of IL-2 in PD-1Ab21-CD19CAR-T cell culture supernatant is about 2 times that in CD19CAR-T cell culture supernatant, and the concentration of interferon-γ (IFN-y) is also significantly higher than that in CD19CAR-T cell culture supernatant.

The above results indicate that the PD-1Ab21-CD19CAR-T cells have stronger anti-tumor function and proliferation ability in the process of recognizing and killing tumor cells. At the same time, they tend to differentiate into memory cells and can resist functional failure induced by the tumor micro-environment.

The present application further provides a preclinical experimental method for treating a B-cell leukemia by PD-1Ab21-CD19CAR-T cells, including:
inoculating the severely immunodeficient NSG mice with 2×10⁵ fluorescently-labeled human lymphoma cells Raji-Luc through tail vein injection, on day 7 post tumor bearing, treating the mice with 5×10⁶ CD19CAR-T or PD-1Ab21-CD19CAR-T cells inoculated through tail vein injection, and periodically monitoring the tumor progression via in-vivo bioluminescence imaging. The results show that compared with CD19CAR-T cells, PD-1Ab21-CD19CAR-T can significantly suppress the progression of B-cell leukemia and even completely eradicated tumors in some cases. The above results indicate that the PD-1Ab21-CD19CAR-T cells exhibit markedly enhanced anti-tumor function in vivo in a humanized mouse model of B-cell leukemia.

The present application further provides a preclinical experimental method for treating a B-cell lymphoma by PD-1Ab21-CD19CAR-T cells, including:
subcutaneously inoculating the severely immunodeficient NSG mice with 5×10⁶ human lymphoma cells Daudi-PDL1, when tumor volume reached 150-250 mm³, treating the tumor-bearing mice with 2×10⁶ CD19CAR-T or PD-1Ab21-CD19CAR-T cells through tail vein injection, respectively, measuring the tumor size periodically, and collecting peripheral blood for analysis of numbers of CAR-T cells by flow cytometry; and at designated time points, harvesting tumors, spleens, and peripheral tissues of mice for analysis of tissue-infiltrating CAR-T cells. The results show that, compared with CD19CAR-T cells, PD-1Ab21-CD19CAR-T cells significantly inhibit the tumor growth and significantly improve the survival rate of tumor-bearing mice. Peripheral-blood monitoring reveals that the proportion of CD3⁺CD45⁺ cells in the PD-1Ab21-CD19CAR-T cell treatment group is significantly higher than in the CD19CAR-T cell treatment group. Analysis results of mice tissues reveal that on day 12 post CAR-T reinfusion, the proportion and proliferative capacity (Ki-67 expression) of CD3⁺CD8⁺T cells in peripheral blood, spleens, and tumor tissues of the PD-1Ab21-CD19CAR-T cell treatment group are all substantially higher than those of the CD19CAR-T cell treatment group. These results indicate that the PD-1Ab21-CD19CAR-T cells exhibit markedly enhanced anti-tumor function in vivo in a humanized mouse model of lymphoma.

The present application further provides a preclinical experimental method for treating a myeloma by PD-1Ab21-CD19CAR-T cells, including:
inoculating the severely immunodeficient NSG mice with 1×10⁷ fluorescently-labeled human myeloma cells U266-Luc through tail vein injection, when tumors become clearly detectable by small animal in vivo imaging, treating the tumor-bearing mice with 2×10⁶ BCMA CAR-T or PD-1Ab21-BCMACAR-T cells through tail vein injection, respectively, and periodically monitoring the tumor progression via in-vivo bioluminescence imaging. The results show that the PD-1Ab21-BCMACAR-T cells can significantly inhibit progression of myeloma compared with BCMACAR-T cells, and even completely eradicate tumors in some cases. The above results indicate that the PD-1Ab21-BCMACAR-T cells exhibit markedly enhanced anti-tumor function in vivo in a humanized mouse model of myeloma.

The present application further provides a preclinical experimental method for treating a Her2/neu⁺ breast cancer by PD-1Ab21-Her2CAR-T cells, including:
to evaluate the therapeutic effect of CAR-T cells expressing and secreting PD-1Ab21 on solid tumors, using a humanized mouse model of Her2/neu⁺ breast cancer for Her2CAR-T cell therapy, subcutaneously inoculating the severely immunodeficient NSG mice with 1×10⁷ of the human Her2/neu⁺ breast cancer cells BT474, when tumor volume reached 150-200 mm³, treating the tumor-bearing mice with 3×10⁶ Her2CAR-T or PD-1Ab21-Her2CAR-T cells through tail vein injection, respectively, and periodically measuring the tumor size. The results show that compared with Her2CAR-T, PD-1Ab21-Her2CAR-T cells can significantly inhibit tumor growth and exhibit a markedly stronger anti-tumor therapeutic effect. These results indicate that CAR-T cells secreting PD-1Ab21 possess the same enhanced in vivo anti-tumor activity against solid tumors compared with conventional CAR-T cells.

Preparation and efficacy validation examples of CAR-T cells will be provided below.

Experimental animals were 6- to 8-week-old female NSG mice purchased from GemPharmatech Co., Ltd.. During experiments, mice were housed in the Immunodeficient Animal Facility of the Institute of Biophysics, Chinese Academy of Sciences. All experimental procedures and mouse processing procedures followed Regulations for the Administration of Affairs Concerning Experimental Animals and were approved by the "Institutional Animal Care and Use Committee (IACUC)" of the Institute of Biophysics.

Cell lines and cell culture used in the examples of the present application included: Human embryonic kidney epithelial cell line 293T, Human breast cancer cell line BT474-PDL1, Human Burkitt's lymphoma cell line Daudi-PD-L1, and Human PBMCs obtained from this laboratory. Mouse B-cell progenitor cell line Baf3 was purchased from Beijing Bena Culture Collection Co., Ltd. 293T cells were cultured in DMEM culture medium (GIBCO) containing 10% inactivated fetal bovine serum (Hyclone Company), and 1% penicillin/streptomycin (Invitrogen Company), BT474-PDL1, Daudi-PDL1 and Baf3 cells were cultured in RPMI-1640 culture medium (GIBCO) containing 10% inactivated fetal bovine serum (Hyclone Company), and 1% penicillin/streptomycin (Invitrogen Company). All cells were incubated in an incubator (5% CO₂) at 37°C.

Antibodies used in the examples of the present application:

**Table 1 Antibodies for detection of human lymphocyte surface markers**

| Names | Clone Nos. | Companies |
|---|---|---|
| Anti-CD8 | OKT8 | Invitrogen |
| Anti-CD4 | OKT4 | Invitrogen |
| Anti-PD-1 | MIH4 | Invitrogen |
| Anti-TIM3 | FAB2365P | R&D |
| Anti-CD3 | UCHL1 | Invitrogen |
| Anti-CD45 | HI30 | Invitrogen |

**Table 2 Antibodies for detection of human lymphocyte intracellular markers**

| Names | Clone Nos. | Companies |
|---|---|---|
| Anti-Ki67 | DG9 | Biolegend |
| Anti-pSTAT3 | | |

Major reagents and kits used in the examples of the present application:

**Table 3 Reagents and Kits**

| Names | Item Nos. | Companies |
|---|---|---|
| 4% Paraformaldehyde | AR-0211 | DINGGUO |
| DMSO | 67-68-5 | SIGMA |
| Coomassie Brilliant Blue R250 | 1.12553 | SIGMA |
| Anti-human CD3 | 16-0037-85 | Invitrogen |
| Anti-huamn CD28 | 16-0289-85 | Invitrogen |
| Human Lymphocyte Separation Medium | LTS1077 | TBD |
| β-Mercaptoethanol | 60-24-2 | SIGMA |

### Example 1: Construction of Fusion Protein, CD19CAR, and PD-1Ab21-CD19CAR

### 1. Construction of Fusion Protein

The hybridoma cells generating anti-human PD-1 antibody were subjected to exon sequencing and engineering modifications to obtain the humanized antibody sequences. Based on the gene sequences of the heavy chain and light chain of the antibody, the following primers were designed to clone the coding genes of the heavy and light chain variable regions of the antibody by PCR. The primer sequences are as follows:
The PCR technology used the following primers separately:
Anti-human PD-1 antibody heavy chain (H) upstream primer:
   ATGACCAGGCTGACAGTGCTG (SEQ ID No.75);
Anti-human PD-1 antibody heavy chain (H) downstream primer:
   CGCTAGACACTGTCACCAGGGTG (SEQ ID No.76);
Anti-human PD-1 antibody light chain (L) upstream primer:
   GACGTGCTGACCCAGAGCCCATC (SEQ ID No.77); and
Anti-human PD-1 antibody light chain (L) downstream primer:
   CACTGTCCTCTTGATCTCCAG (SEQ ID No.78)

After the coding genes of the heavy chain (H) and light chain (L) variable regions were obtained by cloning, a linker sequence (linker1): GGAGGAGGAGGATCTGGAGGAGGAGGAAGCGGAGGAGGAGGATCC, was used to link the coding genes of the heavy chain (H) and light chain (L) variable regions, thereby forming the coding gene sequence of an anti-PD-1 scFv (PD-1Ab).

Further, another linker sequence (linker2) consisted of 15 amino acids: GGAGGAGGAGGAAGCGGAGGAGGAGGATCCGGCGGCGGCGGCTCT, was used to link the obtained anti-PD-1 scFv (PD-1Ab) sequence with the human IL-21 coding gene sequence (SEQ ID No. 15). The coding genes of fusion protein (PD-1Ab21) including the anti-PD-1 scFv and IL-21 were obtained, and the nucleotide sequence of which is shown in SEQ ID No.3, and the specific structure is shown in FIG. 1a.

For the subsequent protein purification and detection, a linker3 consisted of 5 amino acids and coding gene sequence of tag protein 10×His were added to the C-terminus of the obtained fusion protein, the nucleotide sequence of which is shown in SEQ ID No.1, and the specific structure as shown in FIG. 1b.

The primer sequences are as follows:
IL-21 upstream primer sequence
   CAAGGTCAAGATCGCCACATGATTAG (SEQ ID No.79); and
IL-21 downstream primer sequence

CTAGTGATGGTGGTGATGATGGTGGTGATGGTGAGAGCCTCCACCCCCGGAATCTTCACT
TCCGTG (SEQ ID No.80).

### 2. Construction of CD19CAR

The CD19CAR consisted of an extracellular domain, a transmembrane region, and an intracellular region. Extracellular domain fragment was composed of CD8α signal peptide (its nucleotide and amino acid sequences were shown in SEQ ID Nos.23 and 24, respectively), humanized CD19 scFv (its nucleotide and amino acid sequences were shown in SEQ ID No.31 and SEQ ID No.32, respectively), CD8α hinge region (its nucleotide and amino acid sequences were shown in SEQ ID No.33 and SEQ ID No.34, respectively); the transmembrane region was composed of CD8α transmembrane region (its nucleotide and amino acid sequences were shown in SEQ ID No.35 and SEQ ID No.36, respectively); and the intracellular domain was composed of 4-1BB intracellular signaling domain (its nucleotide and amino acid sequences were shown in SEQ ID No.37 and SEQ ID No.38, respectively), and CD3ζ chain intracellular domain sequence (its nucleotide and amino acid sequences were shown in SEQ ID No.39 and SEQ ID No.40, respectively). By linking the extracellular domain, transmembrane region, and intracellular domain of CAR together, the CD19CAR sequence was obtained, and its nucleotide and amino acid sequences were shown in SEQ ID No.43 and SEQ ID No.44, respectively. The specific construction structural schematic diagram is shown in FIG. 1c.

### 3. Construction of PD-1Ab21-CD19CAR

The nucleotide sequence of the obtained fusion protein was linked downstream of the CD19CAR sequence via a P2A sequence (its nucleotide and amino acid sequences were shown in SEQ ID No.41 and SEQ ID No.42, respectively) to obtain the PD-1Ab21-CD19CAR sequence and its nucleotide and amino acid sequences were shown in SEQ ID No.19 and SEQ ID No.20, respectively. The specific construction structural schematic diagram is shown in FIG. 1e.

### 4. Construction of Expression Vector Plasmid

The nucleotide sequences of CD19CAR and PD-1Ab21-CD19CAR (SEQ ID No.43 and SEQ ID No.19, respectively) were inserted separately into the CAR expression vector plasmid, and sequencing was performed to verify whether the sequence was correct.

### Example 2: Preparation of CAR-T Cells and Functional Characterization of Secreted PD-1Ab21 Fusion Protein

### Packaging of CAR Lentivirus

1) On day 1, 293T cell subculture was performed in a 10 cm culture dish 16 to 20 hours before transfection, with 4×10⁶ cells per dish.
2) On day 2, the subcultured cells on day 1 were observed, once 50% of the cell coverage rate was reached, the transfection was performed, first the transfection reagents (2× HEPES salt buffer), the expression vector plasmids constructed in Example 1, and the lentiviral packaging helper plasmids were restored to room temperature. The transfection complex was prepared in a 50 ml centrifugal tube, with one 50 ml centrifugal tube accommodating reagents for up to 50 dishes. The single-dish transfection system and reagent dosage were as follows:
   Solution A: 35 µg of plasmids (15 µg of CAR expression vector plasmids, 7.5 µg of the helper plasmid RRE, 6 µg of REV and VSVG), and 50 µl of 2.5 mol CaCl₂
      ddH₂O up to 500 µl
   Solution B: 500 µl of 2× HEPES

After preparing the solution A, it was allowed to stand for 3 minutes, then added into the solution B (2× HEPES salt buffer), meanwhile, bubbled in the reagent tube with an electronic pipette to thoroughly mix the two solutions rapidly until white turbidity appeared in the reagent tube, which were the calcium phosphate transfection particles, it should be noted that overly rapid mixing resulted in poor formation of calcium phosphate transfection particles, while overly slow mixing resulted in particle aggregation, both conditions will reduce transfection efficiency,
1 ml of the transfection mixture was added to the culture dish containing 293T cells, gently shaken to mix well, and incubated in an incubator at 37°C overnight (12 to 16 hours).
3) On day 3, the culture supernatant was discarded and 15 ml of DMEM culture medium containing 10% FBS was carefully and slowly added along the wall of culture dish.
4) On day 5, the 48-h post-medium-change viral supernatant was collected, and supplemented with 10 ml of a fresh culture medium, then filtered through a 0.45-µm filter and stored at 4°C for 1 day.
5) On day 6, the 72-h post-medium-change viral supernatant was collected, filtered through a 0.45-µm filter, and combined with the 48-h viral culture supernatant and concentrated 50-fold using a concentration pump, after which, it was filtered again through a 0.22-µm filter, aliquoted into a 1.5-ml EP tubes, and stored at -80°C.

### Isolation of PBMCs from Healthy Donors

1) The concentrated human peripheral blood was collected and diluted proportionally, 50 ml of concentrated peripheral blood was diluted with 70 ml of serum-free 1640 culture medium and mixed well;
2) 15 ml of lymphocyte separation medium was added to a 50 ml centrifugal tube, then 30 ml of diluted peripheral blood was carefully added with a pipette along the wall of the centrifugal tube;
3) The centrifugal tube was slowly placed in the centrifuge, acceleration speed of the centrifuge was set to 3 and deceleration speed to 1, then centrifuged at 2200 rpm for 25 minutes;
4) Following centrifugation, the cells in the centrifuge tube were separated into four layers, and the top layer was carefully removed with a pipette;
5) The second layer of lymphocytes was aspirated carefully and transferred into a new 50-ml centrifugal tube, serum-free 1640 culture medium was added to reach a volume of 50 ml, then mixed well, and centrifuged; the acceleration and deceleration speeds were restored to 9 at 1800 rpm for 10 min;
6) The supernatant was discarded, cells were resuspended with serum-free culture medium and centrifuged at 1600 rpm for 6 min, and then centrifugation was repeated once; and
7) The cells were counted and cryopreserved.

### Activation of T Cells from Human Peripheral Blood

1) The cryopreserved cells were taken out from liquid nitrogen, and thawed rapidly at 37°C, the number of cells was counted, the density of cells was adjusted to 1×10⁷/ml, inoculated into a 24-well plate, and incubated in an incubator overnight;
2) On the following day, the number of cells was counted and the required volume of magnetic beads was calculated based on the ratio of 1 µl magnetic beads per 4×10⁵ cells;
3) The cells were activated with anti-CD3 and anti-CD28 magnetic beads; 1 ml of Gibco culture medium was added into a sterile flow tube, then an appropriate volume of magnetic beads was added, the sterile flow tube was placed onto a magnetic groove for about 30 seconds, the culture medium in the sterile flow tube was removed, the cell suspension was added, then gently mixed with the magnetic beads, after processing with a mixer for half an hour, the mixture was transferred to a 24-well plate, and the cells were stood in an incubator overnight; and
4) Lentiviral infection was generally performed 24 hours post activation.

### Preparation of CAR-T Cells

1) T cells activated with CD3/CD28 beads for 24 h were selected, the density was adjusted to 1×10⁶/ml, and 500 µl of cell suspension per well was added in a 48-well plate;
2) The lentiviruses were taken from the freezer at -80°C and allowed it to returned to room temperature, then 500 µl was added to each well;
3) 8 µg/ml of Polybrene and 10 ng/ml of IL-2 were added to each well, mixed well, and the plate was sealed with plastic wrap;
4) Centrifugal infection: centrifuge was performed at 2000 g, 30°C, for 60 min, with acceleration speed set to 9 and deceleration speed to 0, after the centrifugation was completed, the plate was placed into a cell incubator at 37°C;
5) On the following day, 500 µl of culture medium was carefully removed from each well and 500 µl of retrovirus was added, supplemented with Polybrene and IL-2, and centrifugal infection was performed under the same conditions above;
6) Following centrifugation was completed, it was returned to the cell incubator, and 500 µl of culture medium was removed 6 hours later, supplemented with additional CAR-T culture medium and incubated overnight; and
7) On day 3, the cells were collected from each well, 10 ml of CAR-T culture medium was added, centrifuged at 2000 rpm for 10 minutes, the supernatant was discarded, and resuspended to 5×10⁵/ml, the expression of CAR cells (CD19Fc-PE) was detected by flow cytometry (see FIG. 2A), and subsequent procedures were performed according to experimental needs.

### Assessment of the Biological Function of PD-1Ab21

To assess the biological function of anti-PD-1 scFv of PD-1Ab21 protein, a CHO-PD-1 cell line with high surface expression of human PD-1 was used.

### Binding of PD-1Ab21 Protein to Cell Surface PD-1

1) The 48-h culture supernatants of PD-1Ab21CD19CAR-T and CD19CAR-T cells were collected, then the supernatants were concentrated by 100-fold with a 10KD ultrafiltration tube at 2800 rpm (centrifugation time was depended on concentration situations);
2) 1×10⁵ CHO-PD-1 cells were collected and 50 µl of the concentrated supernatant was added, and the cells were mixed well. The cells were incubated at 4°C for 30 min, then 1 ml of 1×PBS was added, centrifuged at 4000 rpm for 3 minutes, and the supernatant was discarded;
3)50 ul of FACS buffer (1 ml fetal calf serum + 49 ml PBS) was added to resuspend the cells, 0.5 ul of anti-His-PE antibody was added to the cell suspension, and incubated at 4°C for 30 minutes, 1 ml 1×PBS was added, centrifuged at 4000 rpm for 3 minutes, and the supernatant was discarded; and
4) 100 ul of PBS was added to resuspend the cells, then passed through a cell strainer, and subjected to flow cytometry analysis.

### Blocking of Binding of PD-1 Antibody to PD-1 on Cell Surface by PD-1Ab21 Protein

1) The 48-h culture supernatants of PD-1Ab21CD19CAR-T and CD19CAR-T cells were collected, then the supernatants were concentrated by 100-fold with a 10KD ultrafiltration tube at 2800 rpm (centrifugation time was depended on concentration situations);
2) 1×10⁵ CHO-PD-1 cells were collected and 50 µl of the concentrated supernatant was added, and the cells were mixed well. The cells were incubated at 4°C for 30 min, then 1 ml of 1×PBS was added, centrifuged at 4000 rpm for 3 minutes, and the supernatant was discarded;
3) 50 ul of FACS buffer (1 ml fetal calf serum + 49 ml PBS) was added to resuspend the cells, 0.5 ul of anti-PD-1-APC antibody was added to the cell suspension, and incubated at 4°C for 30 minutes, 1 ml 1×PBS was added, centrifuged at 4000 rpm for 3 minutes, and the supernatant was discarded; and
4) 100 ul of PBS was added to resuspend the cells, then passed through a strainer, and subjected to flow cytometry analysis.

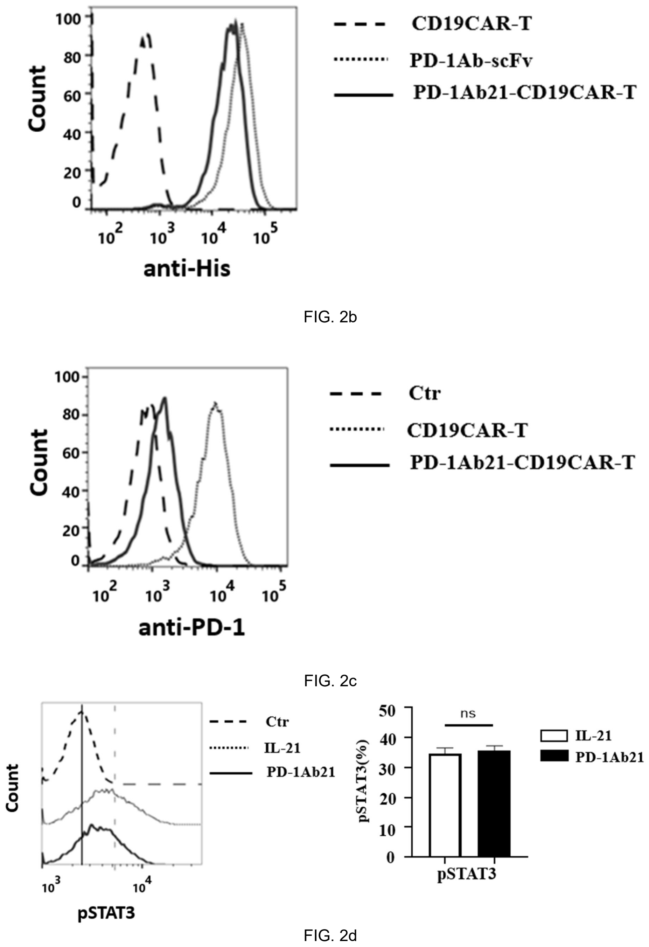

FIG. 2a shows the proportion CAR⁺ cells in the prepared PD-1Ab21-CD19CAR-T cells. After the 48-h culture supernatant of PD-1Ab21-CD19CAR-T cells was added to CHO-PD-1 cells, since the secreted PD-1Ab21 protein by PD-1Ab21-CD19CAR-T cells was labeled with a His tag, His positive signal can be detected using anti-His flow cytometry antibody, indicating the binding of PD-1Ab21 protein to PD-1 molecules (FIG. 2b); when anti-PD-1 antibody was added, the binding of PD-1Ab21 protein to PD-1 molecules blocked the surface binding of anti-PD-1 antibody to PD-1, preventing the detection of anti-PD-1 positive signals (FIG. 2c).

### PD-1Ab21 Protein-Induced STAT3 Phosphorylation in Baf3 Cells

To assess the biological function of the IL-21 portion of PD-1Ab21 protein, human IL-21 (0.3uM) and the 48-h culture supernatant of PD-1Ab21-CD19CAR-T cells were added to Baf3 culture system, respectively to stimulate Baf3 cells, then the effects of human IL-21 and PD-1Ab21 proteins on the phosphorylation of STAT3 at tyrosine 705 (Y705) site were detected.
1) The Baf3 cells were placed in serum-free 1640 medium (Resting) overnight;
2) The overnight-rested Baf3 cells were collected, centrifuged at 1200 rpm for 3 minutes, the supernatant was discarded, the cells were resuspended to 1×10⁶/ml, and plated in a 24-well plate with 500 µl per well;
3) IL-21 (0.3 uM) and 48-h concentrated supernatant of PD-1Ab21-CD19CAR-T cells were added to each well for stimulation, and then incubated at 37°C for 30 minutes;
4) The stimulated Baf3 cells were collected into 500 µl of pre-chilled 4% paraformaldehyde and fixed at 4°C for 30 minutes, then centrifuged at 4000 rpm for 3 minutes and the supernatant was discarded;
5) 1 ml of -20°C pre-chilled methanol was added for permeabilization at 4°C for 30 minutes, then centrifuged at 4000 rpm for 3 minutes, and the supernatant was discarded;
6) 1 ml of FACS buffer was added to resuspend the cells, then centrifuged at 4000 rpm for 3 minutes, and the supernatant was discarded, the above procedures were repeated once;
7)50 ul of FACS was added to resuspend the cells, 0.5 ul of anti-pSTAT3 antibody was added to the cell suspension, and incubated at 4°C for 30 minutes, 1 ml of FACS was added, centrifuged at 4000 rpm for 3 minutes, and the supernatant was discarded; and
8) 100 ul of PBS was added to resuspend the cells, then passed through a cell strainer, and subjected to flow cytometry analysis.

The results show that both IL-21 and PD-1Ab21 can promote the phosphorylation of STAT3 at tyrosine 705 (Y705) in Baf3 cells (FIG. 2D).

### Example 3: In Vitro Proliferation and Phenotypic Characterization of PD-1Ab21-CD19CAR-T Cells

CAR-T cells were prepared according to the method of Example 2, after 48 h of viral infection, the virus was removed, and CD19CAR-T and PD-1Ab21-CD19CAR-T cells were cultured under conditions of 10 U/ml or 100 U/ml of IL-2, respectively.

The results show that under *in vitro* culture conditions with 100 U/ml of IL-2, both types of CAR-T cells exhibited no significant difference in proliferation fold.

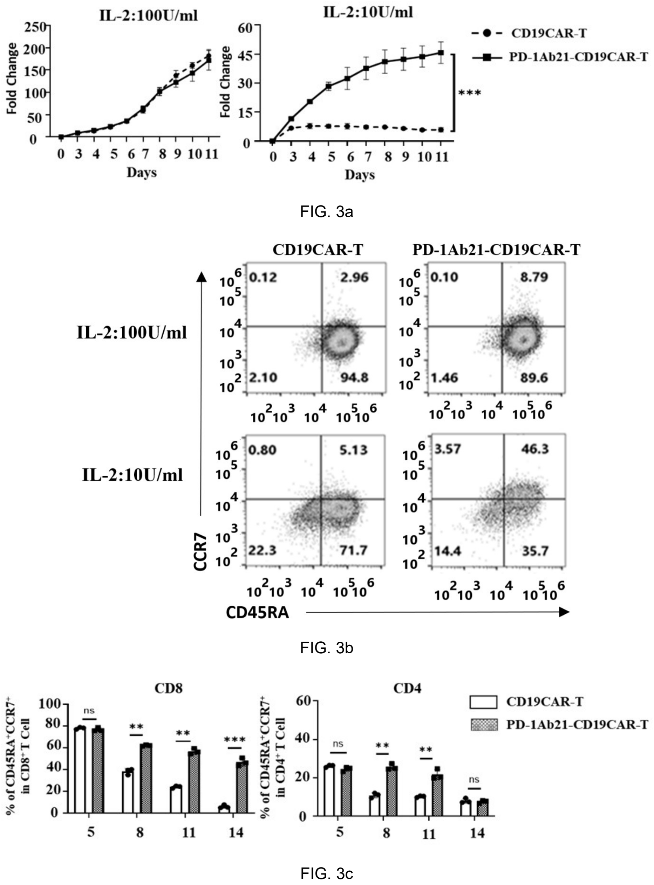

When the concentration of IL-2 was reduced to 10 U/ml during in vitro culture of CAR-T cells, the changes in proliferation times of the two types of CAR-T cells were monitored by cell counting during the in vitro culture process at low concentration of IL-2. The results show that in the in vitro culture system of CAR-T cells with 10 U/ml of IL-2, the CD19CAR-T cells exhibited essentially no proliferation, whereas the PD-1Ab21-CD19CAR-T cells demonstrated continuous proliferative capacity during the first 7 days of culture, indicating markedly stronger proliferation ability compared with CD19CAR-T cells (FIG. 3a).

Further, the changes in phenotype of the two types of CAR-T cells were analyzed by flow cytometry during the in vitro culture process.

### Surface Staining:

1) The isolated cell suspension was collected into a 1.5-ml EP tube at 5×10⁵ cells/sample and resuspended in 100 µl of FACS buffer;
2) The fluorescently-labeled antibody and isotype control antibody were selected, marked at 4°C in the dark for 20 minutes, washed with 1 ml of FACS buffer, and centrifuged at 4000 rpm for 3 minutes; and
3) 100 ul of PBS was added to resuspend the cells, the passed through a cell strainer, and proceeded to flow cytometry analysis.

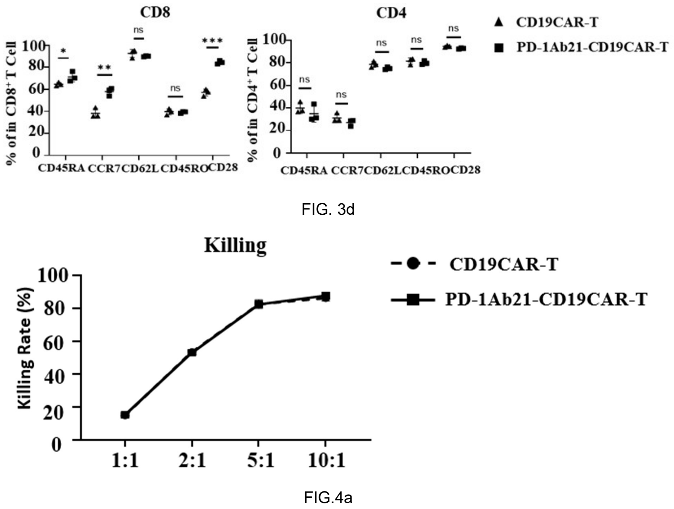

It was found that under conditions of 10 U/ml of IL-2, the proportions of T_{SCM} (CD45RA⁺CCR7⁺) cells in the CD4 or CD8 T cells were calculated at different culture days (FIG. 3b and FIG. 3c), and at the same time, under conditions of 10 U/ml of IL-2, expression levels of CD45RA, CCR7, CD62L, CD45RO, and CD28 in CD4 or CD8 T cells were also calculated at different culture days (FIG. 3d). The results show that the PD-1Ab21 can induce the differentiation of CD19CAR-T cells toward T_{SCM} (CD45RA⁺CCR7⁺) cells and possessed stronger sustained proliferative ability.

### Example 4: In Vitro Cytotoxicity Assessment of PD-1Ab21-CD19CAR-T Cells

To evaluate differences in in vitro killing capacity between PD-1Ab21-CD19CAR-T and CD19CAR-T cells against tumor cells, CAR-T cells were prepared according to the method of Example 2, the CAR-T cells in vitro cultured under conditions of 100 U/ml of IL-2 for five days were co-cultured with target Daudi-PDL1 cells in 96-well plates at E(CAR-T):M(Daudi-PDL1) ratios of 1:1/2:1/5:1/10:1 for 24 hours to detect the killing capacity of CAR-T cells. The results show that the short-term killing capacity against tumor cells of CD19CAR-T and PD-1Ab21-CD19CAR-T cells showed no difference (FIG. 4a).

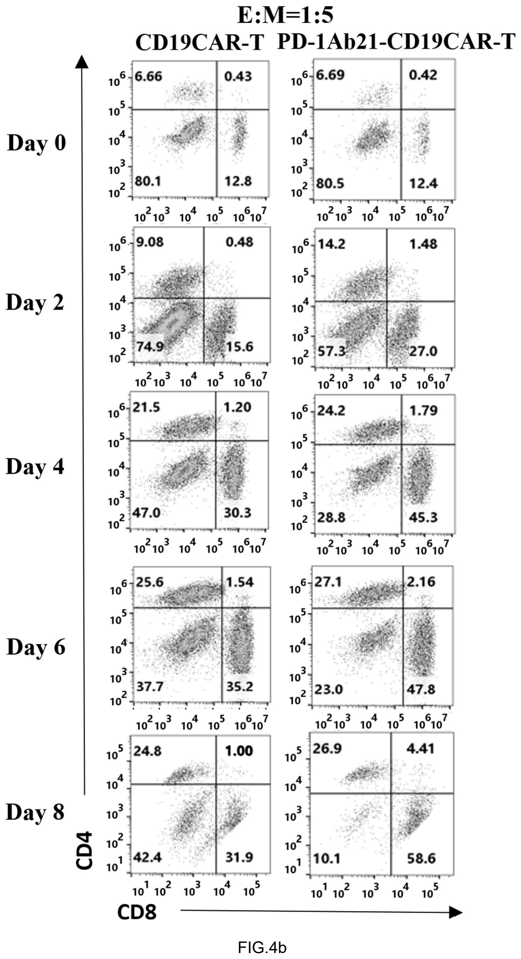

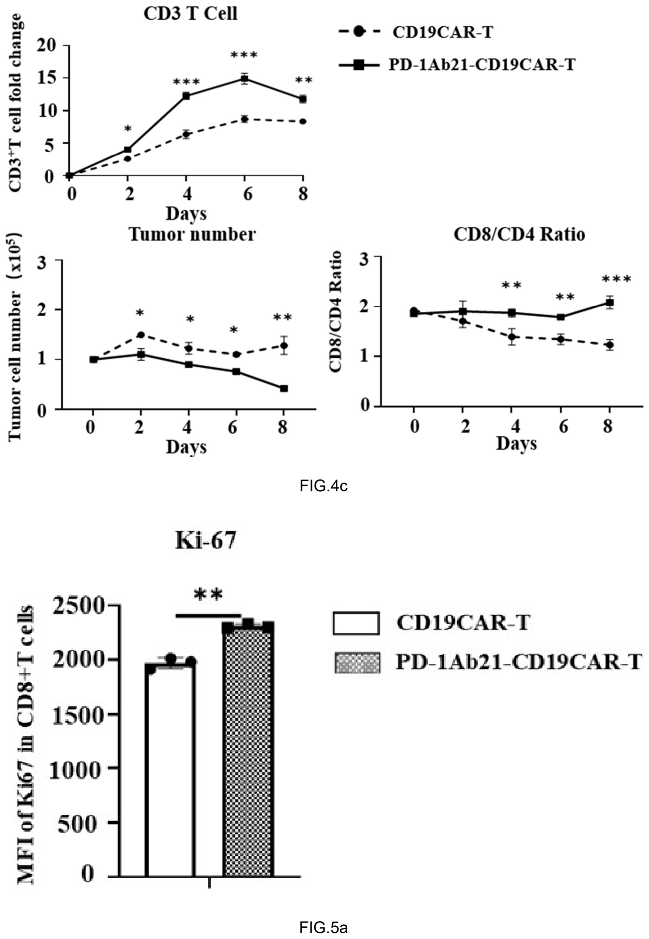

To further evaluate the sustained killing capacity of PD-1Ab21-CD19CAR-T and CD19CAR-T cells against tumor cells, the CAR-T cells in vitro cultured under conditions of 100 U/ml of IL-2 for five days were co-cultured with target Daudi-PDL1 cells at E(CAR-T):M(Daudi-PDL1) ratio of 1:5 for 8 days to detect the sustained killing capacity of CD19CAR-T and PD-1Ab21-CD19CAR-T cells; proportions of T cells and tumor cells were detected by flow cytometry during the sustained killing process (FIG. 4b), and then stained according to the surface staining procedure described in Example 3, at the same time, the numbers of T cells, and tumor cells (Daudi-PDL1), and change in CD8/CD4 ratio were monitored by cell counting (FIG. 4c). The results show that PD-1Ab21-CD19CAR-T cells exhibited significantly enhanced sustained killing capacity compared with CD19CAR-T cells.

### Example 5: Detection of In Vitro Proliferation and Cytokine Secretion Ability of PD-1Ab21-CD19CAR-T Cells

To detect the difference in proliferation and cytokine secretion ability of PD-1Ab21-CD19CAR-T and CD19CAR-T cells post co-culture with tumor cells, CAR-T cells were prepared following the method described in Example 2, the CAR-T cells in vitro cultured for five days under conditions of 100 U/ml of IL-2 were co-cultured with target Daudi-PDL1 cells at an E(CAR-T):M(Daudi-PDL1) ratio of 1:5 for 2 days, to detect the Ki-67 expression of CD19CAR-T and PD-1Ab21-CD19CAR-T cells (FIG. 5a).

### Intranuclear staining for Ki-67:

1) The isolated cell suspension was collected into a 1.5-ml EP tube at 1×10⁶ cells/sample and resuspended in 100 µl of FACS buffer;
2) The surface-labelled antibody were added and marked at 4°C in the dark for 20 minutes; meanwhile isotype control tube was set (with the same number of cells);
3) It was washed twice with 1 ml of FACS buffer;
4) For intranuclear staining of Ki-67 and TCF-1, they were resuspended in 200 µl of Fixation/Permeabilization working solution and incubated at 4°C overnight in the dark and fixed;
5) It was washed twice with 1 ml 1× Permeabilization Buffer, and permeated;
6) The cells were resuspended in 100 µl of Permeabilization Buffer and then anti-Ki-67 and anti-TCF-1 were added, and incubated at 4°C for 30 minutes;
7) It was washed twice with 1 ml Permeabilization Buffer to remove the unbound antibody; and
8) 100 ul of PBS was added to resuspend the cells, then passed through a cell strainer, and subjected to flow cytometry analysis.

The results show that the proliferation marker Ki-67 expression of the CD19CAR-T cell secreting PD-1Ab21 was significantly enhanced, indicating that CD19CAR-T cell secreting PD-1Ab21 had stronger proliferative ability.

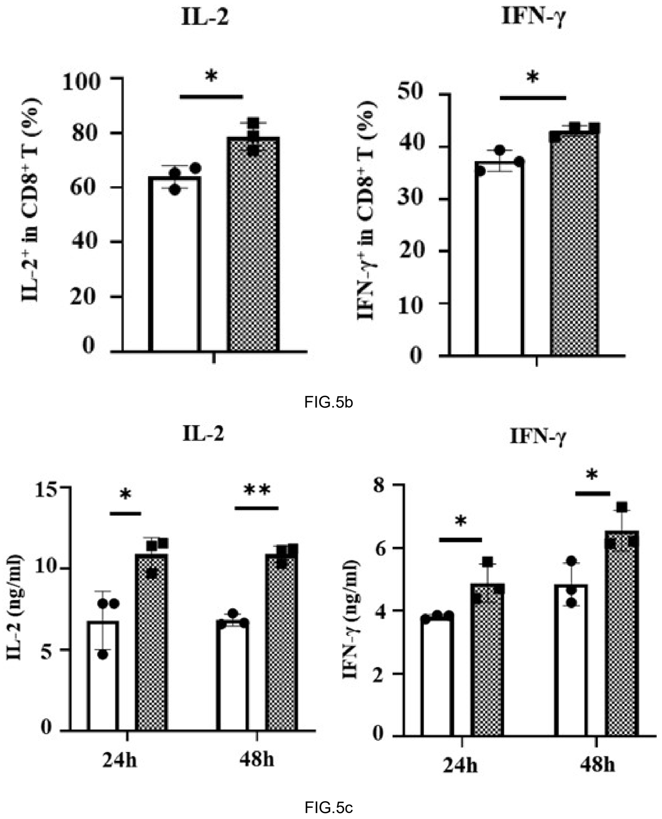

Under the above conditions, the secretion and expression conditions of IL-2 and IFN-γ by CD19CAR-T and PD-1Ab21-CD19CAR-T cells were examined via flow cytometry and ELISA kits (FIG. 5b and FIG. 5c) according to the manufacturer's instructions (Invitrogen: Catalog Number 88-7025). The results show that PD-1Ab21-CD19CAR-T cells exhibited substantially greater cytokine secretion capacity compared with CD19CAR-T cells. This further demonstrates that the PD-1Ab21-CD19CAR-T cells possess significantly enhanced proliferation and cytokine secretion capabilities compared with CD19CAR-T cells.

### Example 6: Exhaustion Phenotype Analysis of PD-1Ab21-CD19CAR-T Cells

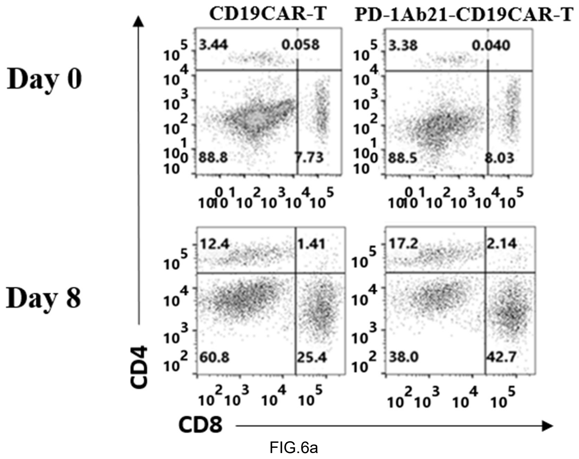

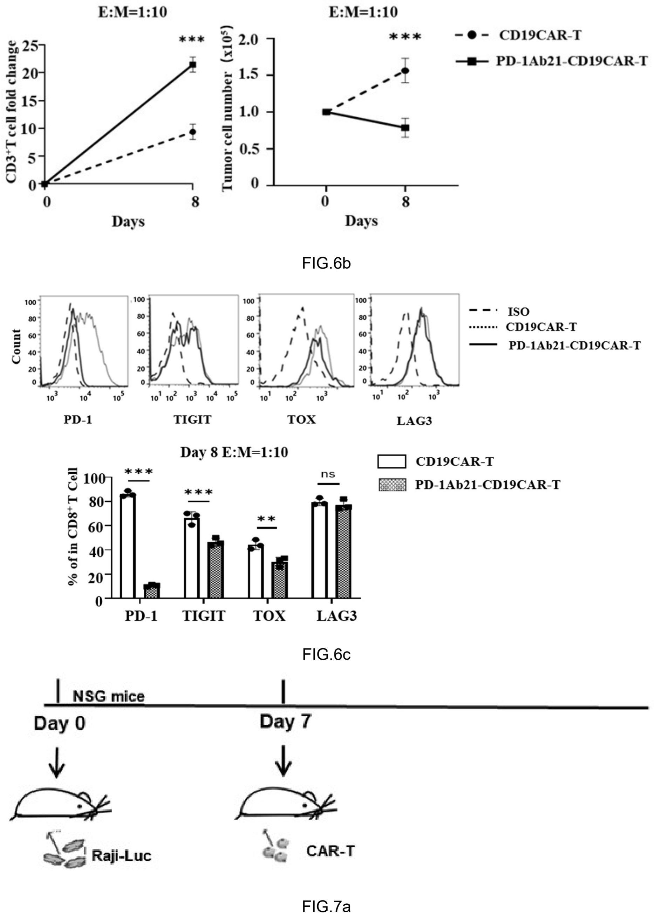

To detect the expression of exhaustion markers of PD-1Ab21-CD19CAR-T and CD19CAR-T cells after they were co-cultured with tumor cells, cell surface staining and intranuclear staining were performed as described in Example 5, the CAR-T cells in vitro cultured under conditions of 100 U/ml of IL-2 for five days were co-cultured with target Daudi-PDL1 cells at E(CAR-T):M(Daudi-PDL1) ratio of 1:10 for 8 days, the changes in proportion and number of CD19CAR-T and PD-1Ab21-CD19CAR-T cells were detected by flow cytometry and cell counting after co-culturing with tumor cells for 8 days (FIG. 6a and FIG. 6b), the results also indicate that PD-1Ab21-CD19CAR-T has significantly stronger proliferation and sustained killing capability against tumor cells compared with CD19CAR-T cells.

The expressions of exhaustion markers PD-1, TIGIT, TOX, and LAG3 on CD8 T cell in CD19CAR-T and PD-1Ab21-CD19CAR-T cells were detected by flow cytometry after 8 days of co-culture with tumor cells (FIG. 6a and FIG. 6b), the results show that the expression of exhaustion markers of PD-1Ab21-CD19CAR-T cells is significantly reduced compared with CD19CAR-T cells (FIG. 6c).

### Example 7: Significant Anti-tumor Efficacy of PD-1Ab21-CD19CAR-T Cells in Humanized Mouse Model of B-cell Leukemia

In this example and Examples 8 and 9 below, the CAR-T cells capable of expressing and secreting a fusion protein without a His tag were constructed and used for animal experiments, and the construction method of which was basically similar to those of Examples 1 and 2. Specifically, firstly, a CAR expression vector was constructed according to the method of Example 1, except that the nucleotide sequence SEQ ID No.1 of the fusion protein PD-1Ab21 was replaced with SEQ ID No.3, the nucleotide sequences of the other domains were shown in Example 1, and the specific construction structure schematic diagram was as shown in FIG. 1d, and the nucleotide and amino acid sequences of the finally constructed PD-1Ab21-CD19CAR were shown in SEQ ID No.21 and SEQ ID No.22, respectively; afterwards, the PD-1Ab21-CD19CAR-T cells were constructed with the CAR expression vector according to the method of Example 2.

To determine whether the constructed PD-1Ab21-CD19CAR-T cells exhibited superior anti-tumor efficacy, studies on the anti-tumor efficacy were first conducted in a humanized mouse model of B-cell leukemia. The severely immunodeficient NSG mice were injected intravenously in tail vein with 2×10⁵ Raji-Luc tumor cells, followed by tail vein reinfusion (i.v.) of 5×10⁶ CD19CAR-T and PD-1Ab21-CD19CAR-T cells of CAR⁺ (FIG. 7a). The small animal in-vivo imaging was performed every 3 days to detect tumor progression, and the method was as follows:

### Small animal imaging

1) 2 mg of Luciferin substrate was injected intraperitoneally into each mouse, and the timing was started;
2) The mice were anesthetized through intraperitoneal injection of tribromoethanol or isoflurane inhalation; and
3) About 5 minutes post injection of sodium fluorescein, the mice were placed in IVIS Lumina3 biological imager, the exposure time was set to 1 minute, and the images were captured for detection.

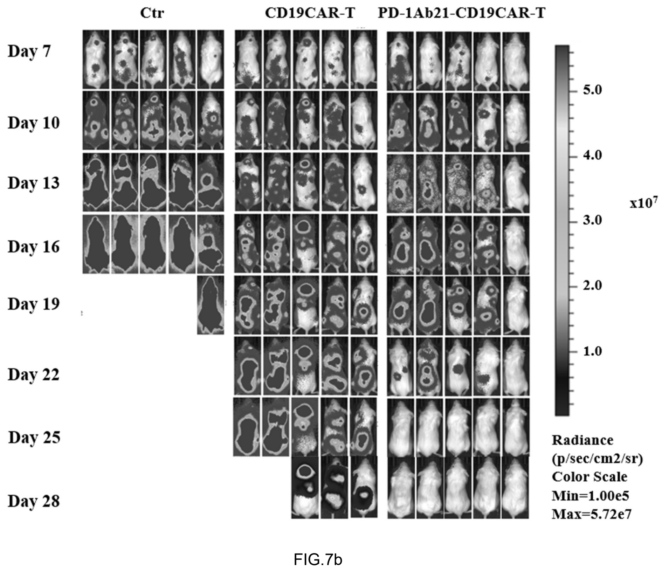

FIG. 7b shows monitoring tumor progression by small animal in vivo imaging; the results show that under high tumor burden, compared with control group, although CD19CAR-T cells were able to partially inhibit leukemia progression, ultimately failed to eradicate the tumor, resulting in mouse mortality; in contrast, PD-1Ab21-CD19CAR-T cells can significantly inhibit tumor progression and completely eradicate the tumor, achieving the effect of curing tumors. These results indicate that the PD-1Ab21-CD19CAR-T cells exhibit markedly superior anti-tumor efficacy compared with CD19CAR-T cells in a humanized mouse model of B-cell leukemia.

### Example 8: Significant Anti-tumor Efficacy of PD-1Ab21-CD19CAR-T Cells in Humanized Mouse Model of Lymphoma

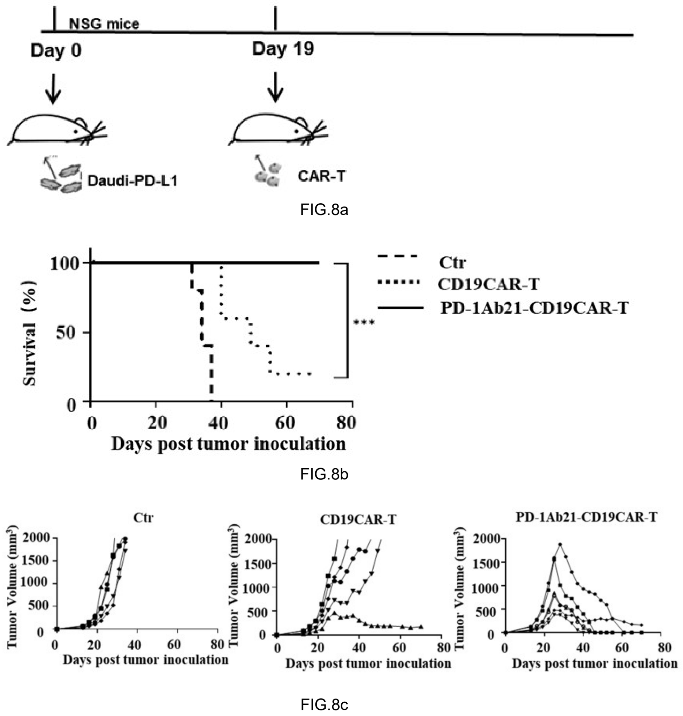

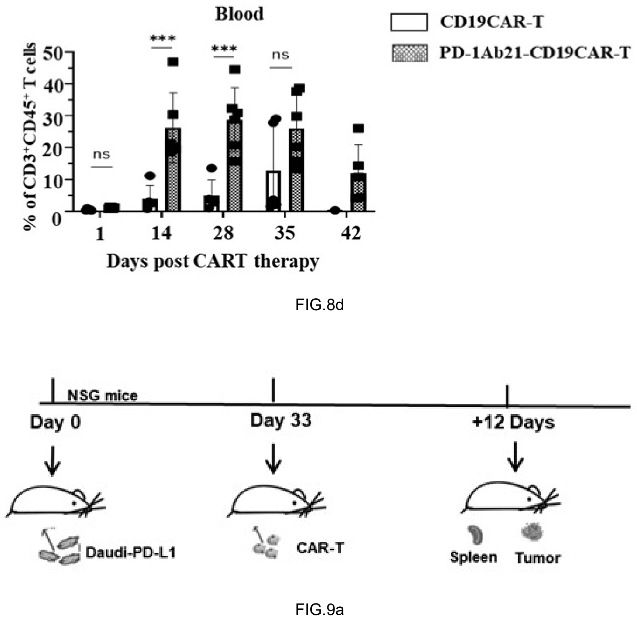

To determine whether the constructed PD-1Ab21-CD19CAR-T cells mediate a stronger anti-tumor efficacy than CD19CAR-T cells in a humanized mouse model of lymphoma, the severely immunodeficient NSG mice were subcutaneously inoculated with 5×10⁶ of Daudi-PDL1 tumor cells, when the tumor volume reached 150-250 mm³, the tumor-bearing mice were treated with 2×10⁶ of CD19CAR-T or PD-1Ab21-CD19CAR-T cells through tail vein injection, respectively, and the tumor size was periodically measured (FIG. 8a). The results showed that compared with CD19CAR-T cells, the PD-1Ab21-CD19CAR-T cells can significantly improve the survival rate of mouse (FIG. 8b), and inhibit the tumor growth (FIG. 8c). By monitoring the change in proportion of CD3⁺CD45⁺CAR-T cells in peripheral blood after treatment, cell surface staining was performed as described in Example 5, and it is observed that the proportion of CD3⁺CD45⁺ CAR-T cells in the peripheral blood was markedly higher in the PD-1Ab21-CD19CAR-T treatment group than that in the CD19CAR-T group (FIG. 8d). Therefore, these results indicate that in the Daudi-PDL1 tumor treatment model, the constructed PD-1Ab21-CD19CAR-T cells exhibit stronger in vivo anti-tumor efficacy compared with CD19CAR-T cells.

### Example 9: Stronger Anti-tumor Efficacy Mediated via PD-1Ab21 by Enhancement of Proliferation and Stem-Like Differentiation of CAR-T Cells

The above results confirmed that the constructed PD-1Ab21-CD19CAR-T cells exhibit stronger in vivo anti-tumor efficacy than CD19CAR-T cells. Next, the mechanism by which the constructed PD-1Ab21-CD19CAR-T cells mediate stronger anti-tumor efficacy was further investigated, and it was found that the proportion of CD3⁺CD45⁺CAR-T cells in the peripheral blood of mice in PD-1Ab21-CD19CAR-T cell treatment group was markedly higher than that of CD19CAR-T cell treatment group (FIG. 8d). This result has caught our attention, it is hypothesized that the fusion protein PD-1Ab21 synthesized and secreted by the constructed PD-1Ab21-CD19CAR-T cells acts on itself in an autocrine or paracrine manner to promote the proliferation of CAR-T cell in vivo, thereby inducing stronger anti-tumor efficacy.

To verify this hypothesis, the Daudi-PDL1 treatment model was used and the peripheral blood, spleen, and tumor tissues, etc., were collected on day 12 post CAR-T cell reinfusion for flow cytometry analysis (FIG. 9a).

### Detection of CAR-T Cell in Peripheral Blood

1) 20 ul of EDTA (50 mM) was pre-added into EP tubes, about 3 drops of blood was collected per mouse;
2) 1 ml of ACK buffer was added to each tube for lysis of erythrocytes until it became clear; then centrifuged at 4000 rpm for 3 minutes. The supernatant was discarded with a pipette tip;
3) The cells were resuspended in 1 ml of FACS buffer (1 ml serum + 49 ml PBS), centrifuged at 4000 rpm for 3 minutes, and the supernatant was discarded with a pipette tip;
4) A flow antibody mix was prepared, 50 ul per sample, according to the number of samples to be tested, two more samples can be mixed and formulated with FACS. Human-specific antibody: 0.5 ul per sample; mouse-specific antibody: 0.25 ul per sample. 50 ul mix was added to each sample, the cells were resuspended with a pipette, and incubated at 4°C for 30 minutes;
5) 1 ml of FACS was added to each sample to wash off the antibody, centrifuged at 4000 rpm for 3 minutes, and the supernatant was discarded; and
6) 100 ul of PBS was added to resuspend the cells, then passed through a cell strainer, and subjected to flow cytometry analysis.

### Preparation of Single-Cell Suspensions from Spleen

1) The mice were sacrificed by cervical dislocation; the spleens were excised and placed in culture dishes containing 1640 culture medium;
2) The tissues were gently dissociated using frosted glass slides; resuspended with culture medium and passed through a 40-µm cell strainer;
3) Then it was centrifuged at 2000 rpm for 10 minutes, and the supernatant was discarded;
4) 1 ml of ACK lysis buffer was added to lyse the erythrocytes, mixed well, and lysed for 5 minutes;
5) 10 ml of PBS was added to stop the lysis; centrifuged at 2000 rpm for 10 minutes; and
6) The supernatant was discarded, and the cells were resuspended in complete 1640 culture medium to prepare the single-cell suspension.

### Preparation of Tumor Single-Cell Suspension

1) The mice were sacrificed by cervical dislocation and the subcutaneous tumor tissues were excised using scissors;
2) The tumor tissues were transferred to a 5-ml EP tube, firstly, cut as small as possible using scissors, then an appropriate volume of triple-enzyme digestion solution was added according to tumor weight, and digested at 37°C for about 20 minutes;
3) The tumor tissues were removed, 10-fold digestive enzyme volume of PBS solution was added to terminate the reaction, and centrifuged at 2000 rpm for 10 minutes; and
4) The cells were resuspended in FACS buffer; passed through a 40-µm cell strainer to prepare a single-cell suspension.

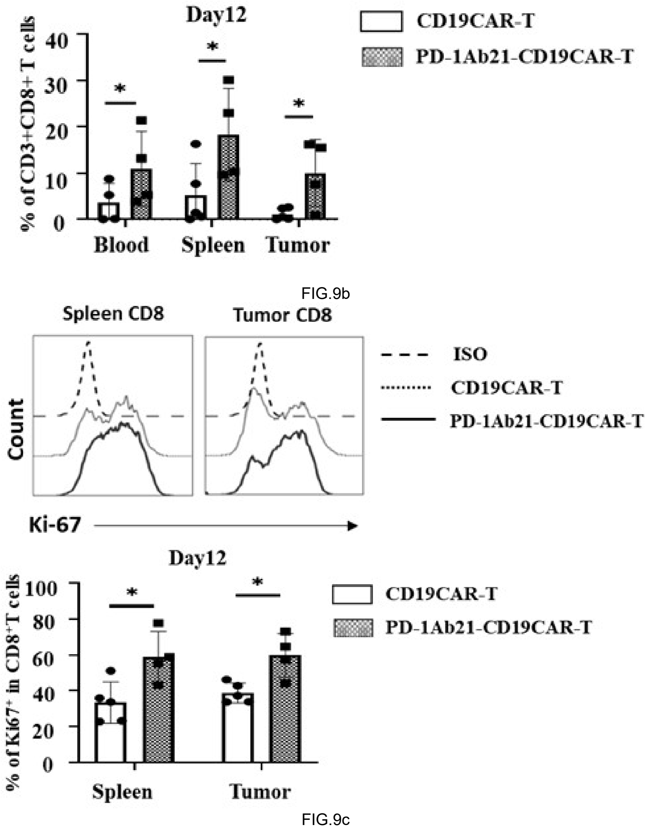

Firstly, the proportion of CAR-T cells reinfused to these sites was detected by flow cytometry, and it was found that the CD8⁺ CAR-T cells mainly exerted the anti-tumor efficacy in vivo. Therefore, the proportions of CD3⁺CD8⁺ CAR-T cells in these sites in the PD-1Ab21-CD19CAR-T cell treatment group and the CD19CAR-T cell treatment group were detected. The results show that the proportion of CD3⁺CD8⁺CAR-T cells in the CD19CAR-T-hPD-1Ab21 cell treatment group was significantly higher than that in the CD19CAR-T cell treatment group (FIG. 9b).

Since Ki-67 is a marker of cell proliferation, the Ki-67 expression of CD8⁺ CART cells in spleen and tumor sites was detected using intranuclear staining as described in Example 5. The results show that the proportion of Ki-67⁺CD8⁺ cells was markedly higher in the PD-1Ab21-CD19CAR-T cell treatment group than that in the CD19CAR-T cell treatment group (FIG. 9c). These results further indicate that the reinfused PD-1Ab21-CD19CAR-T cells had stronger proliferative capacity than the CD19CAR-T cells, and the proliferating CAR-T cells were mainly CD3⁺CD8⁺ CAR-T cells.

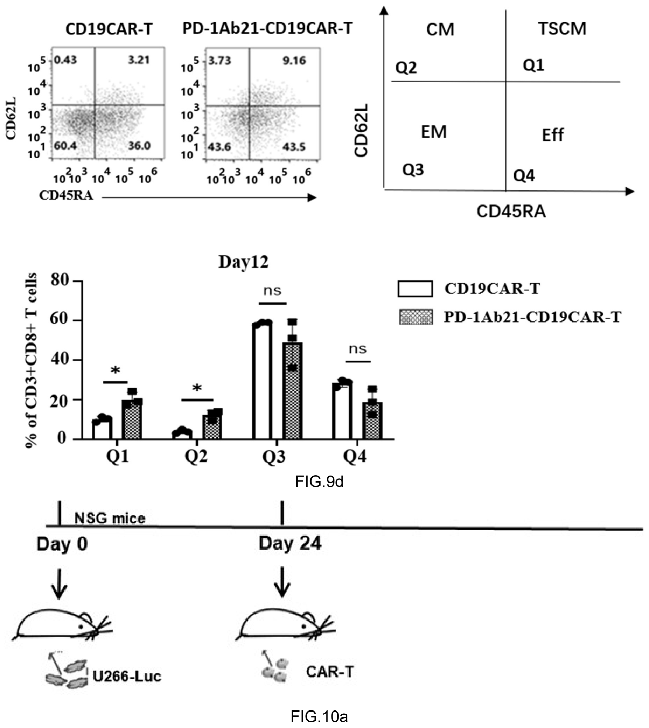

To assess the tumor killing effector function of the effector CD8⁺ T cells at the tumor sites, next the phenotypes of tumor-infiltrating CD3⁺CD8⁺CAR-T cells were detected in the two types of CAR-T cell treatment group using cell surface staining as described in Example 4. Results show that there is no significant difference between the effector CD8⁺T cells of CD45RA⁺CD62L⁻ and the effector memory CD8⁺T cells of CD45RA⁻CD62L⁻ in the two CAR-T treatment groups (FIG. 9d), at the same time, it was observed that the proportions of stem-like memory CD8⁺T cells (CD45RA⁺CD62L⁺) and central memory CD8⁺T cells (CD45RA⁻CD62L⁺) were markedly elevated in the PD-1Ab21-CD19CAR-T treatment group. These results further indicate that the fusion protein PD-1Ab21 secreted by the constructed PD-1Ab21-CD19CAR-T cells promoted the proliferation and stem-like differentiation of CAR-T cells, thereby mediating stronger anti-tumor efficacy.

### Example 10: Significant Anti-tumor Therapeutical Efficacy of PD-1Ab21-BCMACAR-T Cells in Humanized Mouse Model of Myeloma

To evaluate the therapeutic effect of CAR-T cells expressing and secreting PD-1Ab21 on myeloma, a humanized mouse model of myeloma was used for BCMACAR-T cell therapy. The BCMACAR-T and PD-1Ab21-BCMACAR-T cells were constructed according to the methods described in Examples 1 and 2. Specifically, the CAR expression vectors of BCMACAR and PD-1Ab21-BCMACAR were constructed according to the method in Example 1, except that the nucleotide sequence (SEQ ID No.1) of fusion protein PD-1Ab21 was replaced with SEQ ID No. 3, and the nucleotide sequence (SEQ ID No. 31) of the specific scFv targeting CD19 was replaced with the nucleotide sequence (SEQ ID No.45) of the specific scFv targeting BCMA, the nucleotide sequences of the other domains were shown in Example 1, and the specific construction structural schematic diagram is shown in FIG. 1c and FIG. 1d. The nucleotide and amino acid sequences of the finally constructed BCMACAR are shown in SEQ ID No. 53 and SEQ ID No. 54, respectively, and the nucleotide and amino acid sequences of PD-1Ab21-BCMACAR are shown in SEQ ID No. 55 and SEQ ID No. 56, respectively; then the BCMACAR-T cells and PD-1Ab21-BCMACAR-T cells were constructed using the CAR expression vector according to the method of Example 2.

The severely immunodeficient NSG mice were inoculated with 1×10⁷ of U266-Luc myeloma cells in tail vein, followed by tail vein (i.v.) reinfusion of 2×10⁶ of BCMACAR-T and PD-1Ab21-BCMACAR-T cells of CAR⁺ (FIG. 10a). The small animal in vivo imaging was performed every week to detect tumor progression, and the specific method was in accordance with Example 7.

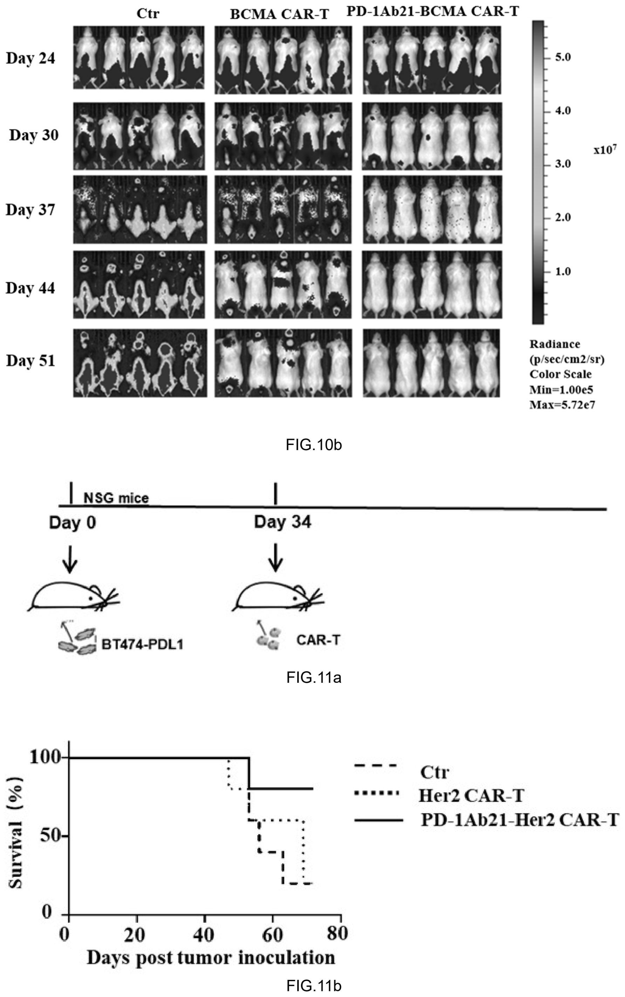

The results show that, compared with control group, the BCMACAR-T cells can inhibit the myeloma progression after reinfusion but were unable to eliminate the tumor burden. In contrast, the PD-1Ab21-BCMACAR-T cells can rapidly inhibit the tumor progression within one week after reinfusion and completely eradicate the tumor burden, achieving the cure of tumors (FIG. 10b). These results show that the PD-1Ab21-BCMACAR-T cells exhibit significant anti-tumor efficacy in a humanized mouse model of myeloma.

### Example 11: Significant Anti-tumor Efficacy of PD-1Ab21-Her2CAR-T Cells in Humanized Mouse Model of Breast Cancer

To evaluate the therapeutic effect of CAR-T cells expressing and secreting PD-1Ab21 on solid tumors, using a humanized mouse model of Her2/neu⁺ breast cancer for Her2CAR-T cell therapy.

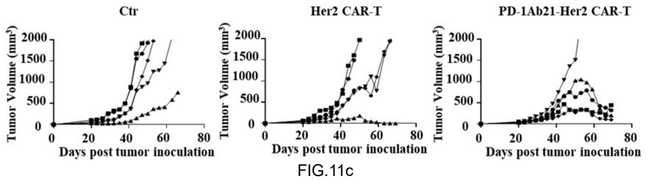

The Her2CAR-T and PD-1Ab21-Her2CAR-T cells were constructed according to the methods of Examples 1 and 2. Specifically, the CAR expression vectors of Her2CAR and PD-1Ab21-Her2CAR were constructed according to the method in Example 1, except that the nucleotide sequence (SEQ ID No.1) of fusion protein PD-1Ab21 was replaced with SEQ ID No. 3, and the nucleotide sequence (SEQ ID No. 31) of the specific scFv targeting CD19 was replaced with the nucleotide sequence (SEQ ID No.57) of the specific scFv targeting Her2, the nucleotide sequences of the remaining domains were shown in Example 1, and the specific construction structural schematic diagram is shown in FIG. 1c and FIG. 1d. The nucleotide and amino acid sequences of the finally constructed Her2CAR are shown in SEQ ID No. 65 and SEQ ID No. 66, respectively, and the nucleotide and amino acid sequences of PD-1Ab21-Her2CAR are shown in SEQ ID No. 67 and SEQ ID No. 68, respectively; then the Her2CAR-T cells and PD-1Ab21-Her2CAR-T cells were constructed using the CAR expression vector according to the method of Example 2. The severely immunodeficient NSG mice was subcutaneously inoculated with 1×10⁷ human Her2/neu⁺ breast cancer cells BT474, when tumor volume reaches 150-200 mm³, treating the tumor-bearing mice with 3×10⁶ of Her2CAR-T or PD-1Ab21-Her2CAR-T cells through tail vein injection, respectively, and periodically measuring the tumor size (FIG. 11a). The results show that compared with Her2CAR-T, PD-1Ab21-Her2CAR-T cells can significantly inhibit tumor growth and exhibit a markedly stronger anti-tumor therapeutic effect (FIG. 11b). The tumor length (a) and width (b) were measured, and the tumor volume was calculated as (ab²/2) (FIG. 11c). These results indicate that CAR-T cells secreting PD-1Ab21 possess the same enhanced in-vivo anti-tumor activity against solid tumors compared with conventional CAR-T cells.

The examples of the present application aim to protect an immune cell, an expression vector, a use and a preparation method thereof, and have the following technical effects:
1. The immune cells (PD-1Ab21-CAR-T cells, PD-1Ab21-TCR-T cells, PD-1Ab21-CAR-NK cells, and PD-1Ab21-TIL cells) of the present application, in addition to exerting the anti-tumor efficacy of a common immune cell, is capable of expressing and secreting the fusion protein PD-1Ab21 including the humanized anti-PD-1 scFv and the IL-21. The secreted fusion protein can block the PD-1 inhibitory signals on CAR-T cells, while simultaneously targeting the IL-21 to the immune cell to promote the proliferation of the immune cell and differentiation into a memory cell, which can significantly enhance the anti-tumor efficacy of the immune cell and improve the therapeutic efficacy against tumors.
2. Since cytokines act on numerous types of target cells, their systemic administration results in substantial adverse effects, greatly limiting their use in disease treatment. The fusion protein PD-1Ab21 secreted by the immune cells of the present application can target the PD-1⁺ anti-tumor T cells in vivo, reducing off-target effects on other cells and greatly mitigating the systemic adverse effects of the cytokine IL-21.

It should be understood that the specific embodiments described above are intended solely to illustrate or explain the principles of the present application and do not constitute limitations. Therefore, any modifications, equivalent substitutions, or improvements, etc., made without departing from the spirit and scope of the present application shall be included within the scope of protection of the present application. Moreover, the appended claims of the present application aim to cover all variations and modifications falling within the scope and boundaries of the claims, or equivalents of such scope and boundaries.

## Claims

1. A fusion protein, wherein the fusion protein comprises an anti-programmed cell death protein 1 (PD-1) single-chain variable fragment (scFv) and interleukin-21 (IL-21).

2. The fusion protein according to claim 1, wherein the anti-PD-1 scFv comprises a heavy chain variable region and a light chain variable region; the heavy chain variable region comprises heavy chain complementarity-determining region 1 (HCDR1), HCDR2, and HCDR3, comprise or consist of amino acid sequences of SEQ ID No.69, SEQ ID No.70, and SEQ ID No.71, respectively; the light chain variable region comprises light chain complementarity-determining region 1 (LCDR1), LCDR2, and LCDR3, which comprise or consist of amino acid sequences of SEQ ID No.72, SEQ ID No.73, and SEQ ID No.74, respectively; and the IL-21 comprises or consists of an amino acid sequence of SEQ ID No.16.

3. An immune cell, wherein the immune cell is capable of expressing and secreting the fusion protein according to claim 1 or 2.

4. The immune cell according to claim 3, wherein the immune cell specifically recognizes at least one of a hematologic malignancy, a lung cancer, a breast cancer, a liver cancer, a pancreatic cancer, a renal cancer, a prostate cancer, an ovarian cancer, a gastrointestinal tumor, a brain tumor, a neuroendocrine tumor, a bone tumor, and a soft tissue tumor.

5. The immune cell according to claim 3 or 4, wherein the immune cell is selected from at least one of chimeric antigen receptor T (CAR-T) cells, T cell receptor-gene engineered T (TCR-T) cells, chimeric antigen receptor natural killer (CAR-NK) cells, and tumor-infiltrating lymphocytes (TILs).

6. A viral vector, wherein the viral vector is capable of expressing the fusion protein according to claim 1 or 2.

7. The viral vector according to claim 6, wherein the anti-PD-1 scFv comprises a heavy chain variable region and a light chain variable region; the heavy chain variable region comprises HCDR1, HCDR2, and HCDR3, which comprise or consist of amino acid sequences of SEQ ID No.69, SEQ ID No.70, and SEQ ID No.71, respectively; the light chain variable region comprises LCDR1, LCDR2, and LCDR3, which comprise or consist of amino acid sequences of SEQ ID No.72, SEQ ID No.73, and SEQ ID No.74, respectively; and the IL-21 comprises or consists of an amino acid sequence of SEQ ID No.16.

8. The viral vector according to claim 6 or 5, wherein after the viral expression vector infects a cell, it causes the cell to secrete the fusion protein according to claim 1 or 2.

9. An immune cell expression vector, wherein the immune cell expression vector comprises an expression plasmid targeting a specific antigen, a porcine teschovirus-1 2A peptide (P2A) sequence fragment, and a coding sequence fragment of the fusion protein according to claim 1 or 2;
the expression plasmid targeting a specific antigen comprises an extracellular domain, a transmembrane domain, and an intracellular domain;
the extracellular domain comprises a CD8α signal peptide fragment, a specific antibody fragment, and a CD8α hinge region fragment;
the transmembrane domain comprises a CD8α transmembrane region fragment; and
the intracellular domain comprises a 4-1BB signaling domain fragment and a CD3ζ signaling domain fragment.

10. The immune cell expression vector according to claim 9, wherein the anti-PD-1 scFv comprises a heavy chain variable region and a light chain variable region; the heavy chain variable region comprises HCDR1, HCDR2, and HCDR3, which comprise or consist of amino acid sequences of SEQ ID No.69, SEQ ID No.70, and SEQ ID No.71, respectively; the light chain variable region comprises LCDR1, LCDR2, and LCDR3, which comprise or consist of amino acid sequences of SEQ ID No.72, SEQ ID No.73, and SEQ ID No.74, respectively; and the IL-21 comprises or consists of an amino acid sequence of SEQ ID No.16.

11. The immune cell expression vector according to claim 9 or 8, wherein a complementary deoxyribonucleic acid (cDNA) sequence of the coding sequence fragment of the fusion protein according to claim 1 or 2 is shown in SEQ ID No.1 or SEQ ID No.3.

12. The immune cell expression vector according to any one of claims 9 to 11, wherein the specific antibody fragment comprises a specific scFv targeting CD19, and/or a specific scFv targeting a B-cell maturation antigen (BCMA), and/or a specific antibody fragment targeting a Her2/neu tumor antigen.

13. A use of an immune cell in treating a B-cell leukemia, a B-cell lymphoma, a myeloma, a Her2/neu⁺ breast cancer, and an advanced B-cell malignancy, wherein the immune cell is the immune cell according to any one of claims 3 to 5.

14. A preparation method of the immune cell according to any one of claims 3 to 5, wherein the method comprises steps of:
constructing a coding gene of the fusion protein according to claim 1 or 2;
constructing an immune cell expression vector expressing the fusion protein according to claim 1 or 2;
performing virus packaging on the immune cell expression vector to obtain viral particles; and
preparing the immune cell with the viral particles.

15. The preparation method according to claim 14, wherein the step of constructing a coding gene of the fusion protein according to claim 1 or 2 comprises:
selecting a humanized PD-1 antibody and linking a heavy chain variable region with a light chain variable region together via Linker1 to obtain an anti-PD-1 scFv targeting specific cells; and
linking the anti-PD-1 scFv targeting specific cells with a cDNA sequence of IL-21 cytokine via Linker2 and performing DNA fragment synthesis to obtain the coding gene of the fusion protein according to claim 1 or 2.

16. The preparation method according to claim 14, wherein the step of constructing an immune cell expression vector expressing the fusion protein according to claim 1 or 2 comprises:
constructing an expression plasmid targeting a specific antigen with a lentiviral vector pCDH, wherein the expression plasmid targeting a specific antigen comprises an extracellular domain, a transmembrane domain, and an intracellular domain;
the extracellular domain comprises a CD8α signal peptide fragment, a specific antibody fragment, and a CD8α hinge region fragment;
the specific antibody fragment comprises a specific scFv targeting CD19, and/or a specific scFv targeting BCMA, and/or a specific antibody fragment targeting a Her2/neu tumor antigen;
the transmembrane domain comprises a CD8α transmembrane region fragment; and
the intracellular domain comprises a 4-1BB signaling domain fragment and a CD3ζ signaling domain fragment;
linking a coding sequence fragment of the fusion protein according to claim 1 or 2 downstream of a coding fragment of the expression plasmid targeting a specific antigen via a P2A sequence fragment; and
optionally, attaching a His tag to a C-terminus of an entire sequence to obtain the immune cell expression vector expressing the fusion protein according to claim 1 or 2.

17. The preparation method according to claim 14, wherein the step of performing virus packaging on the immune cell expression vector to obtain viral particles comprises:
performing the virus packaging with a calcium phosphate transfection method;
16 to 20 hours before transfection, selecting 293T cells for subculture and inoculating 4×10⁶ 293T cells into each cell culture dish;
20 to 24 hours post inoculation, observing the subculture of the 293T cells and performing the transfection when a coverage rate of the 293T cells reaches 50%; wherein
the transfection method comprises: preparing a transfection reagent comprising solution A and solution B, wherein the solution A comprises 35 µg of plasmids, 50 µl of 2.5 mol CaCl₂, and ddH₂O up to 500 µl, the plasmids comprises 15 µg of CAR expression vector plasmids, 7.5 µg of a helper plasmid Rev-response element (RRE), 6 µg of a regulator of expression of viral proteins (REV), and 6 µg of vesicular stomatitis virus G glycoproteins (VSVG), and the solution B comprises 500 µl of 2× HEPES salt buffer; after the solution A is prepared, standing it for 3 minutes, and then adding the solution A to the solution B while bubbling in a reagent tube with an electronic pipette to mix the solution A and the solution B evenly until the solution in the reagent tube becomes white and turbid, thereby preparing the transfection reagent; and adding 1 ml of the transfection reagent to the culture dish containing the 293T cells, gently shaking to mix evenly, and placing the culture dish in an incubator at 37°C for transfection;
12 to 16 hours post the transfection, performing a medium change by discarding culture supernatant and adding 15 ml of Dulbecco's modified Eagle's medium (DMEM) medium containing 10% fetal bovine serum (FBS);
48 hours post the medium change, filtering the solution through a 0.45-µm filter, collecting 48-hour culture supernatant, making a supplement with 10 ml of DMEM medium containing 10% FBS, and storing it at 4°C;
24 hours post the storage, filtering the solution through a 0.45-µm filter and collecting 72-hour culture supernatant; and
concentrating both the 48-hour culture supernatant and the 72-hour culture supernatant by 50-fold with a concentration pump, then filtering the solution through a 0.22 µm filter, collecting the filtered virus concentrate and dispensing it to a 1.5-mL Eppendorf (EP) tube, and then storing it at -80°C to obtain the viral particles.

18. The preparation method according to claim 14, wherein the step of preparing the immune cell with the viral particles comprises:
retrieving the viral particles and reobtaining a virus concentrate;
collecting human anticoagulant peripheral blood, diluting it with an equal volume of phosphate buffer saline (PBS), and separating the diluted human anticoagulant peripheral blood with a human lymphocyte separation medium to collect peripheral blood mononuclear cells (PBMCs);
adjusting a concentration of T cells in the PBMCs to 1×10⁷/ml, adding anti-CD3 and anti-CD28 magnetic beads to sort the T cells, and activating them to bring the T cells into an activated state;
24 hours post the activation of the T cells, inoculating the T cells into a 24-well plate at a density of 5×10⁵ cells/mL, and adding the virus concentrate and 1 ml of an infection culture medium for virus infection;
6 hours post the infection, making a supplement with 1 ml of a fresh infection culture medium;
continuing the infection for 48 hours, discarding culture supernatant, and replacing it with a fresh infection culture medium; and
continuing the infection to expand the T cells for 6 to 8 days to obtain the immune cell.
